(19) Europäisches Patentamt
European Patent Office
Office européen des brevets

(11) **EP 3 568 024 B1**

(12) **EUROPEAN PATENT SPECIFICATION**

(45) Date of publication and mention
of the grant of the patent:
**25.11.2020 Bulletin 2020/48**

(21) Application number: **18704863.2**

(22) Date of filing: **12.01.2018**

(51) Int Cl.:
*A23C 9/12* *(2006.01)*          *A23C 9/123* *(2006.01)*
*C12R 1/225* *(2006.01)*          *C12R 1/46* *(2006.01)*
*C12N 9/38* *(2006.01)*

(86) International application number:
**PCT/EP2018/050708**

(87) International publication number:
**WO 2018/130630 (19.07.2018 Gazette 2018/29)**

(54) **PROCESS FOR PRODUCING A FERMENTED MILK PRODUCT**

VERFAHREN ZUR HERSTELLUNG EINES FERMENTIERTEN MILCHPRODUKTS

PROCÉDÉ DE PRODUCTION D'UN PRODUIT LAITER FERMENTÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.01.2017 EP 17151378**

(43) Date of publication of application:
**20.11.2019 Bulletin 2019/47**

(73) Proprietor: **Chr. Hansen A/S
2970 Hoersholm (DK)**

(72) Inventors:
• **BA, Zhaoyong
  2980 Kokkedal (DK)**
• **BUCHHORN, Gaelle Lettier
  2830 Virum (DK)**
• **BULDO, Patrizia
  2400 Copenhagen NV (DK)**
• **HOEGHOLM, Tina
  2980 Kokkedal (DK)**
• **RUNGE, Mette Oehrstroem
  3070 Snekkersten (DK)**
• **SCHOELER, Jeppe
  8000 Aarhus C (DK)**
• **VOJINOVIC, Vojislav
  3230 Graested (DK)**

(56) References cited:
**WO-A1-2007/021204     WO-A1-2009/071539
WO-A1-2015/193449     WO-A1-2015/193459
US-A1- 2009 088 391     US-A1- 2015 086 675**

**Description**

FIELD OF THE INVENTION

The present invention relates to a process for producing a fermented milk product.

BACKGROUND OF THE INVENTION

WO 2009/071539 discloses a lactase originating from *Bifidobacterium bifidum,* which is capable of very efficient hydrolysis in milk, and which is active over a broad pH range, including low pH, e.g. a pH below 5. The lactase may be used in processes for producing milk and fermented milk products, such as cheese, yogurt, butter, butter milk, sour cream etc., for reducing the content of lactose.

WO 2013/160413 discloses a method of producing a fermented milk product using a combination of glucose-negative lactic acid bacteria strains and a conventional lactase with an object of reducing the content of lactose in the fermented milk product while increasing the content of glucose.

EP-A1 -2 957 180 in one embodiment discloses a method of producing a fermented milk product using a combination of a starter cultures and a conventional lactase with an object of reducing content of lactose and the level of post-acidification in the fermented milk product. EP-A1-2 957 180 in a second embodiment discloses a method of producing a fermented milk product using lactose-deficient lactic acid bacteria.

SUMMARY OF THE INVENTION

The object of the present invention is to provide an improved process for producing a fermented milk product.

The object of the present invention is obtained by a process for producing a fermented milk product comprising the steps of

1) adding a starter culture comprising at least one lactic acid bacteria strain to a milk base,
2) fermenting the milk for a period of time until a target pH is reached,
3) wherein the starter culture comprises at least one lactose-deficient strain, which is capable of metabolizing a non-lactose carbohydrate, and
4) adding a low pH stable lactase to the process either at the start, during or at the end of the fermentation step, wherein the low pH stable lactase retains its activity at a pH of 5.0 and a temperature of 37 °C at a level of at least 5 % as compared to its activity at the optimum pH of the lactase.

Lactose-deficient lactic acid bacteria typically grow on a non-lactose carbohydrate source, such as sucrose, galactose and glucose, added to the milk in an amount measured so as stop the fermentation process and the growth of the lactic acid bacteria by depletion of the added carbohydrate source. Hereby, the post-acidification during subsequent storage is lowered significantly or even fully prevented. A low pH stable lactase will be active during the full course of a fermentation process and hence allows conversion of most or all of the lactose present in milk to glucose and galactose. Hereby, it is possible to produce a fermented milk product with a reduced content of lactose or a lactose-free product. Also, it is possible to produce a fermented milk product with an increased natural sweetness, as glucose and galactose has a much higher sweetness than lactose.

The present invention is based on the recognition that by using a combination of a low pH stable lactase and lactose-deficient lactic acid bacteria, it is possible to obtain a fermented milk product, which at the same time has reduced lactose content, an increased sweetness and reduced post-acidification. Furthermore, it has surprisingly been found that the said combination results in a fermented milk product having an increased texture as compared to using lactose-deficient lactic acid bacteria and no lactase and as compared to using a low pH stable lactase and lactose-positive lactic acid bacteria.

Furthermore, when the lactase is added before the fermentation step the process of the invention has provided a possibility of reducing or even eliminating the amount of non-lactose carbohydrate to be added to the milk. Thus, the glucose and galactose formed by the enzymatic action of lactase will be available for the growth of the lactose-deficient lactic acid bacteria.

Finally, when the lactase is added after the fermentation step, it is possible to take full advantage of the ability of the lactose-deficient lactic acid bacteria to avoid post-acidification.

Worldwide, a significant numbers of consumers are intolerant or sensitive to lactose. Therefore, there is presently a high demand for dairy products, including fermented milk products, with a reduced content of lactose or which is substantially free of lactose. The present invention has provided a new approach for producing such product in a simple and cost-efficient manner.

DETAILED DISCLOSURE OF THE INVENTION

**Lactase**

The lactase of the fermented milk product of the invention may be any lactase, which retains its activity at a pH of 5.0 and a temperature of 37 °C at a level of at least 5 % as compared to its activity at the optimum pH of the lactase.

In relation to the present invention the activity in LAU of the lactase is measured as specified in the "Definitions" section below.

In a preferred embodiment of the invention, the lactase retains its activity at a pH of 5.0 and a temperature of 37 °C at a level of at least 10 %, preferably at least 20 %, more preferably at least 30 %, more preferably at least 40 %, more preferably at least 50 %, more preferably at least 60 %, more preferably at least 70 %, and most preferably at least 80 %, as compared to its activity at the optimum pH of the lactase.

In a preferred embodiment of the invention, the lactase retains its activity at a pH of 4.0 and a temperature of 37 °C at a level of at least 5 %, preferably at least 10 %, more preferably at least 20 %, more preferably at least 30 %, more preferably at least 40 %, more preferably at least 50 %, more preferably at least 60 %, more preferably at least 70 %, and most preferably at least 80 %, as compared to its activity at the optimum pH of the lactase.

In a preferred embodiment of the invention, the lactase retains its activity at a pH of 3.0 and a temperature of 37 °C at a level of at least 5 %, preferably at least 10 %, more preferably at least 20 %, more preferably at least 30 %, more preferably at least 40 %, more preferably at least 50 %, more preferably at least 60 %, more preferably at least 70 %, and most preferably at least 80 %, as compared to its activity at the optimum pH of the lactase.

In connection with the present invention the optimum pH of the lactase is determined by measuring the lactase activity at pH using the method indicated in the "Definitions" section below and determining the pH with optimum activity. In a preferred embodiment of the invention, the lactase retains its activity at a temperature of 10 °C and a pH of 6.0 at a level of at least 10 % as compared to its activity at the optimum temperature of the lactase. Preferably, the lactase retains its activity at a temperature of 10 °C and a pH of 6.0 at a level of at least 20 %, more preferably at least 30 %, more preferably at least 40 %, more preferably at least 50 %, more preferably at least 60 %, more preferably at least 70 %, and most preferably at least 80 %, as compared to its activity at the optimum temperature of the lactase.

In connection with the present invention the optimum temperature of the lactase is determined by measuring the lactase activity at different temperatures using the method indicated in the "Definitions" section below and determining the temperature with optimum activity.

In a preferred embodiment, the lactase to be used in the product of the present invention has a lactase activity at 37°C and pH 5 which is at least 55%, such as at least 60%, at least 65%, at least 70% or at least 75%, of its lactase activity at 37°C and pH 6.

In another preferred embodiment, the lactase to be used in the product of the present invention has a lactase activity at 37°C and pH 4.5 which is at least 10%, such as at least 20%, at least 30%, at least 35% or at least 40%, of its lactase activity at 37°C and pH 6.

In another preferred embodiment, the lactase to be used in the product of the present invention has a pH optimum of the lactase activity at 37°C which is above pH 5.5.

In another preferred embodiment, the lactase to be used in the product of the present invention has a lactase activity at a temperature of 52°C and a pH of 6.5 which is at least 50%, such as at least 55%, at least 60%, at least 65%, at least 70%, at least 75% or at least 80%, of its lactase activity at a temperature of 38°C and a pH of 6.5.

In a preferred embodiment of the present invention, $K_m$ of the lactase at 5°C is below 25 mM, such as below 20 mM, below 15 mM or below 10 mM. In another preferred embodiment, $K_m$ of the lactase at 37°C is below 25 mM, such as below 20 mM or below 15 mM. The skilled person will know how to determine $K_m$ for the lactase activity at a specific temperature. $K_m$ may be determined by the method described in WO2009/071539.

In another preferred embodiment, the enzyme when hydrolyzing the lactose in the milk product has a ratio of lactase to transgalactosylase activity of more than 1:1, such as more than 2:1 or more than 3:1. In another preferred embodiment, the enzyme treatment is performed under conditions where the lactase activity of the enzyme is higher than the transgalactosylase activity, such as at least two times higher or at least three times higher.

The ratio of lactase to transgalactosylase activity in the milk product may, e.g., be determined by HPLC analysis. In another preferred embodiment, the enzyme treatment is performed under conditions where at least 50% (w/w%) of the hydrolyzed lactose is converted into free galactose. In another preferred embodiment, the enzyme treatment is performed under conditions where the hydrolyzed lactose is converted into equal amounts of free glucose and free galactose.

A lactase in the context of the present invention is a glycoside hydrolase having the ability to hydrolyze the disaccharide lactose into constituent galactose and glucose monomers. The group of lactases, to which the lactase of the invention belongs, comprises but is not limited to enzymes assigned to subclass EC 3.2.1.108. Enzymes assigned to other subclasses, such as, e.g., EC 3.2.1.23, may also be lactases in the context of the present invention. A lactase in the context of the invention may have other activities than the lactose hydrolyzing activity, such as for example a transgalactosylating activity. In the context of the invention, the lactose hydrolyzing activity of the lactase may be referred to as its lactase activity or its beta-galactosidase activity.

Enzymes having lactase activity to be used in a method of the present invention may be of animal, of plant or of microbial origin. Preferred lactases are obtained from microbial sources, in particular from a filamentous fungus or yeast, or from a bacterium.

The enzyme may, e.g., be derived from a strain of *Agaricus,* e.g. *A. bisporus; Ascovaginospora; Aspergillus,* e.g. *A. niger, A. awamori, A. foetidus, A. japonicus, A. oryzae; Candida; Chaetomium; Chaetotomastia; Dictyostelium,* e.g. *D. discoideum; Kluveromyces,* e.g. *K. fragilis, K. lactis; Mucor,* e.g. *M. javanicus, M. mucedo, M. subtilissimus; Neurospora,* e.g. *N. crassa; Rhizomucor,* e.g. *R. pusillus; Rhizopus,* e.g. *R. arrhizus, R. japonicus, R. stolonifer; Sclerotinia,* e.g. *S. libertiana; Torula; Torulopsis; Trichophyton,* e.g. *T. rubrum; Whetzelinia,* e.g. *W. sclerotiorum; Bacillus,* e.g. *B. coagulans, B. circulans, B. megaterium, B. novalis, B. subtilis, B. pumilus, B. stearothermophilus, B. thuringiensis; Bifidobacterium,* e.g. *B. longum, B. bifidum, B. animalis; Chryseobacterium; Citrobacter,* e.g. *C. freundii; Clostridium,* e.g. *C. perfringens; Diplodia,* e.g. *D. gossypina; Enterobacter,* e.g. *E. aerogenes, E. cloacae Edwardsiella, E. tarda; Erwinia,* e.g. *E. herbicola; Escherichia,* e.g. *E. coli; Klebsiella,* e.g. *K. pneumoniae; Miriococcum; Myrothesium; Mucor; Neurospora,* e.g. *N. crassa; Proteus,* e.g. *P. vulgaris; Providencia,* e.g. *P. stuartii; Pycnoporus,* e.g. *Pycnoporus cinnabarinus, Pycnoporus sanguineus; Ruminococcus,* e.g. *R. torques; Salmonella,* e.g. *S. typhimurium; Serratia,* e.g. *S. liquefasciens, S. marcescens; Shigella,* e.g. *S. flexneri; Streptomyces,* e.g. *S. antibioticus, S. castaneoglobisporus, S. violeceoruber; Trametes; Trichoderma,* e.g. *T. reesei, T. viride; Yersinia,* e.g. *Y. enterocolitica.*

In a preferred embodiment, the lactase originates from a bacterium, e.g. from the family Bifidobacteriaceae, such as from the genus *Bifidobacterium,* such as from a strain of *B. bifidum, B. animalis* or *B. longum.* In a more preferred embodiment, the lactase originates from *Bifidobacterium bifidum.*

In a preferred embodiment, an enzyme having lactase activity to be used in the product of the present invention comprises an amino acid sequence which is at least 50% identical to a sequence selected from the group consisting of amino acids 28-1931 of SEQ ID NO: 1, amino acids 28-1331 of SEQ ID NO: 2, SEQ ID NO: 3, SEQ ID NO: 4 and lactase active fragments thereof. In a more preferred embodiment, the enzyme comprises an amino acid sequence which is at least 60%, such as at least 70%, at least 80%, at least 90%, at least 95% or at least 98% identical to a sequence selected from the group consisting of amino acids 28-1931 of SEQ ID NO: 1, amino acids 28-1331 of SEQ ID NO: 2, SEQ ID NO: 3, SEQ ID NO: 4 and lactase active fragments thereof.

A preferred enzyme is a lactase having a sequence which is at least 50%, such as at least 60%, at least 70%, at least 80%, at least 90%, at least 95% or at least 98% identical to amino acids 28-1931 of SEQ ID NO: 1 or to a lactase active fragment thereof. Such lactase active fragment of SEQ ID NO: 1 may be any fragment of SEQ ID NO: 1 having lactase activity. A lactase active fragment of SEQ ID NO: 1 may be, e.g., amino acids 28-979, amino acids 28-1170, amino acids 28-1323, amino acids 28-1331, or amino acids 28-1600 of SEQ ID NO: 1.

In a preferred embodiment, an enzyme having lactase activity to be used in the product of the present invention comprises an amino acid sequence which is at least 50% identical to amino acids 28-1331 of SEQ ID NO: 2. In a more preferred embodiment, the enzyme comprises an amino acid sequence which is at least 60%, such as at least 70%, at least 80%, at least 90%, at least 95% or at least 98% identical to amino acids 28-1331 of SEQ ID NO: 2.

In another embodiment, an enzyme having lactase activity to be used in product of the present invention has an amino acid sequence which is at least 50% identical to SEQ ID NO: 3. Preferably, the enzyme has an amino acid sequence which is at least 60%, such as at least 70%, at least 80%, at least 90%, at least 95% or at least 98% identical to SEQ ID NO: 3.

In another embodiment, an enzyme having lactase activity to be used in the product of the present invention has an amino acid sequence which is at least 50% identical to SEQ ID NO: 4. Preferably, the enzyme has an amino acid sequence which is at least 60%, such as at least 70%, at least 80%, at least 90%, at least 95% or at least 98% identical to SEQ ID NO: 4.

For purposes of the present invention, the degree of identity between two amino acid sequences is determined using the Needleman-Wunsch algorithm (Needleman and Wunsch (1970) J. Mol. Biol. 48: 443-453) as implemented in the Needle program of the EMBOSS package (EMBOSS: The European Molecular Biology Open Software Suite, Rice et al. (2000) Trends in Genetics 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labelled "longest identity" (obtained using the -no brief option) is used as the percent identity and is calculated as follows:

(Identical Residues x 100)/(Length of Alignment – Total Number of Gaps in Alignment)

A particular commercial lactase suitable for use in the present invention is Lactase F "Amano" 100SD available from Amano Enzyme Europe.

Lactases to be used in a method of the present invention may be extracellular. They may have a signal sequence at their N-terminus, which is cleaved off during secretion.

Lactases to be used in a method of the present invention may be derived from any of the sources mentioned herein. The term "derived" means in this context that the enzyme may have been isolated from an organism where it is present natively, i.e. the identity of the amino acid sequence of the enzyme are identical to a native enzyme. The term "derived" also means that the enzymes may have been produced recombinantly in a host organism, the recombinantly produced enzyme having either an identity identical to a native enzyme or having a modified amino acid sequence, e.g. having one or more amino acids which are deleted, inserted and/or substituted, i.e. a recombinantly produced enzyme which is a mutant and/or a fragment of a native amino acid sequence. Within the meaning of a native enzyme are included natural variants. Furthermore, the term "derived" includes enzymes produced synthetically by, e.g., peptide synthesis. The term "derived" also encompasses enzymes which have been modified e.g. by glycosylation, phosphorylation etc., whether *in vivo* or *in vitro.* With respect to recombinantly produced enzyme the term "derived from" refers to the identity of the enzyme and not the identity of the host organism in which it is produced recombinantly.

The lactase may be obtained from a microorganism by use of any suitable technique. For instance, a lactase enzyme preparation may be obtained by fermentation of a suitable microorganism and subsequent isolation of a lactase preparation from the resulting fermented broth or microorganism by methods known in the art. The lactase may also be obtained by use of recombinant DNA techniques. Such method normally comprises cultivation of a host cell transformed with a recombinant DNA vector comprising a DNA sequence encoding the lactase in question and the DNA sequence being operationally linked with an appropriate expression signal such that it is capable of expressing the lactase in a culture medium under conditions permitting the expression of the enzyme and recovering the enzyme from the culture. The DNA sequence may also be incorporated into the genome of the host cell. The DNA sequence may be of genomic, cDNA or synthetic origin or any combinations of these, and may be isolated or synthesized in accordance with methods known in the art.

Lactases to be used in a method of the present invention may be purified. The term "purified" as used herein covers lactase enzyme protein essentially free from insoluble components from the production organism. The term "purified" also covers lactase enzyme protein essentially free from insoluble components from the native organism from which it is obtained. Preferably, it is also separated from some of the soluble components of the organism and culture medium from which it is derived. More preferably, it is separated by one or more of the unit operations: filtration, precipitation, or chromatography.

Accordingly, the enzyme having lactase activity may be purified, viz. only minor amounts of other proteins being present. The expression "other proteins" relate in particular to other enzymes. The term "purified" as used herein also refers to removal of other components, particularly other proteins and most particularly other enzymes present in the cell of origin of the lactase. The lactase may be "substantially pure", i.e. free from other components from the organism in which it is produced, i.e., e.g., a host organism for recombinantly produced lactase. Preferably, the lactase is an at least 40% (w/w) pure enzyme protein preparation, more preferably at least 50%, 60%, 70%, 80% or even at least 90% pure.

The term enzyme having lactase activity includes whatever auxiliary compounds that may be necessary for the enzyme's catalytic activity, such as, e.g., an appropriate acceptor or cofactor, which may or may not be naturally present in the reaction system.

The enzyme may be in any form suited for the use in question, such as, e.g., in the form of a dry powder or granulate, a non-dusting granulate, a liquid, a stabilized liquid, or a protected enzyme.

**Lactose-deficient lactic acid bacteria**

The terms "deficiency in lactose metabolism" and "lactose deficient" are used in the context of the present invention to characterize LAB which either partially or completely lost the ability to use lactose as a source for cell growth or maintaining cell viability. Respective LAB are capable of metabolizing one or several carbohydrates selected from sucrose, galactose and/or glucose or another fermentable carbohydrate. Since these carbohydrates are not naturally present in milk in sufficient amounts to support fermentation by lactose deficient mutants, it is necessary to add these carbohydrates to the milk. Lactose deficient and partially deficient LAB can be characterized as white colonies on a medium containing lactose and X-Gal.

In a particular embodiment of the invention, the lactose-deficient strain is capable of metabolizing a non-lactose carbohydrate selected from the group consisting of sucrose, galactose and glucose, preferably sucrose. In a particular embodiment of the invention, the lactose-deficient strain is capable of metabolizing galactose.

In a particular embodiment of the invention, the lactose-deficient strain is selected from the group consisting of lactose-deficient *Streptococcus thermophilus* and lactose-deficient *Lactobacillus delbrueckii* subsp. *bulgaricus.*

In a particular embodiment of the invention, the lactose-deficient strain is selected from the group consisting of:

(a) a *Streptococcus thermophilus* strain, which strain is:

(i) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28952;
(ii) or a strain derived from DSM 28952, wherein the derived strain is further characterized as having the ability to generate white colonies on a medium containing lactose and X-Gal;

(b) a *Streptococcus thermophilus* strain, which strain is:

(i) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28953;
(ii) or a strain derived from DSM 28953, wherein the derived strain is further characterized as having the ability to generate white colonies on a medium containing lactose and X-Gal;

(c) a *Lactobacillus delbrueckii* ssp. *bulgaricus* strain, which strain is:

(i) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28910;
(ii) or a strain derived from DSM 28910, wherein the derived strain is further characterized as having the ability to generate white colonies on a medium containing lactose and X-Gal.

In a particular embodiment of the invention, the starter culture contains both at least one lactose-deficient *Streptococcus thermophilus* and at least one lactose-deficient *Lactobacillus delbrueckii* subsp. *bulgaricus.*

In a particular embodiment of the invention, the starter culture contains both at least one *Streptococcus thermophilus* and at least one *Lactobacillus delbrueckii* subsp. *bulgaricus,* and wherein all *Streptococcus thermophilus* and all *Lactobacillus delbrueckii* subsp. *bulgaricus* strains are lactose-deficient.

## Steps of the process of the invention

In a particular embodiment of the invention, the fermentation step is terminated by a method selected from the group consisting of 1) acidification of the fermented milk rendering at least one strain of the starter culture unable to grow, 2) cooling treatment and 3) depletion of the non-lactose carbohydrate.

In a particular embodiment of the invention, non-lactose carbohydrate is added to the milk base at the start of the fermentation step.

Preferably, the non-lactose carbohydrate is added to the milk base in an amount measured so as to become depleted and hence result in stopping the growth of lactic acid bacteria and in stopping the fermentation. Preferably, the non-lactose carbohydrate is added to the milk base in an amount measured so as to become depleted and hence result in stopping the growth of lactic acid bacteria and in stopping the fermentation at a selected target pH value.

The amount of non-lactose carbohydrate to be added to the milk base depends on a number of parameters, including the lactic acid bacteria strains used in the starter culture, the composition of the milk base, the fermentation temperature and the desired target pH. Also, the amount of non-lactose carbohydrate to be added to the milk base may depend on the type and amount of lactase used in the process. The amount of non-lactose carbohydrate to be added to the milk base can be determined by experimentation, and it is well within the skills of a skilled person to carry out such experimentation.

In a particular embodiment of the invention, the added non-lactose carbohydrate is selected from the group consisting of sucrose, galactose and glucose, preferably sucrose.

In a first aspect of the invention, the low pH stable lactase is added to the milk base at the start of the fermentation step. In this aspect the added lactase will convert the lactose of the milk base to glucose and galactose, which will be available for metabolization for the starter culture in addition to the added non-lactose carbohydrate. In this case, it will not be possible to stop the fermentation by depletion of the added non-lactose carbohydrate. Thus, in a particular embodiment of the first aspect of the invention, the fermentation step is terminated by a method selected from the group consisting of 1) acidification of the fermented milk rendering at least one strain of the starter culture unable to grow, and 2) cooling treatment.

In a particular embodiment of the first aspect of the invention no non-lactose carbohydrate is added to the fermentation step and at least one lactose-deficient lactic acid strain of the starter culture is capable of metabolizing a carbohydrate selected from the group consisting of glucose and galactose. In this embodiment the lactose-deficient lactic acid strain of the starter culture grows solely on the glucose and/or galactose formed by the enzymatic action of the low pH lactase. In this embodiment of the invention, the fermentation is stopped at a target pH value by a method selected from the group consisting of 1) acidification of the fermented milk rendering at least one strain of the starter culture unable to grow, 2) cooling treatment and 3) depletion of the glucose and/or galactose formed by the low pH stable lactase.

In a second aspect of the invention, the low pH stable lactase is added to the fermented milk at the end of the fermentation. In this case, the lactase-containing fermented milk product is preferably stored at a temperature of at least 2 °C for at least 1 day. In a particular embodiment of the process of the invention, the lactase-containing fermented milk product is stored for at least two days, preferably at least 3 days, more preferably at least 4 days, more preferably at least 5 days, more preferably at least 6 days, and most preferably at least 7 days.

In a particular embodiment of the second aspect of the invention, the fermentation step is terminated by depletion of the non-lactose carbohydrate.

In a particular embodiment of the invention the target pH is between 3.2 and 4.8, more preferably between 3.6 and 4.6, more preferably between 3.8 and 4.5 and most preferably between 4.0 and 4.4.

In a particular embodiment of the invention the fermentation temperature is between 35 °C and 45 °C, preferably between 37 °C and 43 °C, and more preferably between 40 °C and 43 °C.

In another particular embodiment of the invention the fermentation is between 15 °C and 35 °C, preferably between 25 °C and 35 °C, and more preferably between 30 °C and 33 °C.

In a particular embodiment of the invention, the fermented milk product is packaged at a temperature between 15 and 45 °C.

In a particular embodiment of the invention, the pH value of the fermented milk product is maintained within a range of 0.3 pH units, preferably within a range of 0.2 pH units and most preferably within a range of 0.1 pH units, when stored after termination of the fermentation at the temperature used for fermentation over a period of 20 hours.

In a particular embodiment of the invention, the amount of added non-lactose carbohydrate is from 1 mg/g to 30 mg/g, preferably from 2 mg/g to 20 mg/g, and more preferably from 3 mg/g to 10 mg/g milk base.

In a particular embodiment of the invention, the amount of added non-lactose carbohydrate is from 0.1 % to 10 %, preferably from 0.2 % to 8 %, preferably from 0.3 % to 2 %, preferably from 0.4 % to 1.5 %, and more preferably from 0.5 % to 1.2 %, wherein % is (w/w) based on milk base.

The starter culture may have the strain composition of any conventional starter culture of lactic acid bacteria, including single strain culture and culture blends, used for producing a specific type of fermented milk product. Other useful bacteria, which may be added to the product in addition to the starter culture, include the probiotic bacteria *Bifidobacterium spp.*

In a particular embodiment of the invention, the fermented milk product after fermentation is subjected to a heat treatment so as to reduce the level of bacteria of the starter culture to no more than 1X10exp02 CFU per g fermented milk product. In this case a particular embodiment is characterized in that the lactase is added after the heat treatment. In this case the lactase-containing fermented milk product is stored at a temperature of at least 2 °C for at least 1 day. In a particular embodiment of the process of the invention, the lactase-containing fermented milk product is stored for at least two days, preferably at least 3 days, more preferably at least 4 days, more preferably at least 5 days, more preferably at least 6 days, and most preferably at least 7 days.

The heat treatment so as to reduce the level of bacteria of the starter culture to no more than 1.0X10exp02 CFU per g fermented milk is preferably carried out by subjecting the starter culture fermented milk product to a temperature of between 50 °C and 110 °C, preferably between 50 °C and 100 °C, preferably between 50 °C and 90 °C, preferably between 60 °C and 85 °C, more preferably between 65 °C and 82 °C, and most preferably between 70 °C and 80 °C. The heat treatment is preferably carried out for a period of between 5 seconds and 180 seconds, preferably between 5 seconds and 120 seconds, more preferably between 5 seconds and 90 seconds, more preferably between 5 seconds and 60 seconds, more preferably between 8 seconds and 50 seconds and most preferably between 10 and 40 seconds. Preferably, the level of bacteria of the starter culture is reduced to no more than 1.0X10exp01 CFU per g fermented milk, more preferably 0 CFU per g. Fermented milk products subjected to a heat treatment so as to reduce the level of bacteria to no more than 1X10exp02 CFU per g are suitable for use storage at ambient temperature, such as storage at a temperature of at least 5 °C, preferably at least 10 °C, more preferably at least 15 °C, and most preferably at least 20 °C.

The low pH stable lactase is added in a suitable amount to achieve the desired degree of lactose hydrolysis under the chosen reaction conditions. In a particular embodiment of the invention, lactase is added in an amount of between 100 and 20000 LAU per liter milk base, preferably between 100 and 10000 LAU per liter milk base, preferably between 100 and 5000 LAU per liter milk base, preferably less than 3000, such as less than 1500, less than 1000, less than 750 or less than 500, LAU per liter milk base.

In a preferred embodiment, the lactase is added at a concentration of between 5 and 400 LAU per g lactose in the milk base, preferably between 5 and 200 LAU per g lactose in the milk base, preferably between 5 and 100 LAU per g lactose in the milk base, preferably less than 50, such as less than 40, less than 30, less than 20 or less than 10, LAU per g lactose in the milk base.

In a preferred embodiment of the invention, the lactase is added to the milk base in an amount of between 2.0 mg/ml and 50 mg/ml, preferably between 5 mg/ml and 48 mg/ml, more preferably between 10 mg/ml and 46 mg/ml, and most preferably between 20 mg/ml and 45 mg/ml.

In a preferred embodiment of the invention, the residual level of lactose at the end of fermentation is less than 40 mg/ml, preferably less than 35 mg/ml, more preferably less than 30 mg/ml, more preferably less than 25 mg/ml, more preferably less than 20 mg/ml, more preferably less than 15 mg/ml, more preferably less than 10 mg/ml, more preferably less than 5 mg/ml, more preferably less than 3 mg/ml, and most preferably less than 1.5 mg/ml.

In a preferred embodiment of the invention, the milk base at the start of the fermentation step has a content of lactose of between 30.0 mg/ml and 70 mg/ml, preferably between 35 mg/ml and 65 mg/ml, more preferably between 40 mg/ml and 60 mg/ml, and most preferably between 50 mg/ml and 60 mg/ml.

In a preferred embodiment of the invention, wherein the low pH-stable lactase is added to the process at the end of the fermentation step, the fermented milk product, to which the lactase is to be added, has a viscosity, which allows easy distribution of the lactase in the fermented milk product, e.g. by mixing.

In a preferred embodiment of the process of the invention, the lactase to be added to the process is provided in a sterile formulation. In another preferred embodiment of the process of the invention the lactase is added to the process under aseptic conditions, e.g. by sterile filtration of a solution of the lactase.

## Fermented milk product

The present disclosure further relates to a fermented milk product produced by the process of the invention.

In a particular aspect the fermented milk product produced by the process of the invention comprises the starter culture of step 3) of the said process and the low pH stable lactase added in step 4) of the said process.

In a particular aspect the fermented milk product is a product, which may be produced using a lactic acid bacteria strain selected from the group consisting of lactose-deficient *Streptococcus thermophilus* and lactose-deficient *Lactobacillus delbrueckii* subsp. *bulgaricus.*

In a particular aspect the fermented milk product is selected form the group consisting of yogurt, cream cheese, sour milk, sour cream, buttermilk, fermented whey, cultured milk, Smetana, Kefir, drinking yogurt, and Yakult. Preferably, the yogurt is selected from the group consisting of set yogurt, stirred yogurt and drinking yogurt.

In a preferred aspect the fermented milk product contains a further food product selected from the group consisting of fruit beverage, fermented cereal products, chemically acidified cereal products, soy milk products and any mixture thereof.

The fermented milk product typically contains protein in a level of between 2.0 % by weight to 3.5 % by weight. The fermented milk product may also be a low protein product with a protein level of between 1.0 % by weight and 2.0 % by weight. Alternatively, the fermented milk product may be a high protein product with a protein level of above 3.5 % by weight.

## Use

The present invention further relates to the use in a process for producing a fermented milk product comprising the steps of

1) adding a starter culture comprising at least one lactic acid bacteria strain to a milk base, and
2) fermenting the milk for a period of time until a target pH is reached, of
3) the starter culture comprising at least one lactose-deficient strain, which is capable of metabolizing a non-lactose carbohydrate, and
4) a low pH stable lactase added to the process either at the start, during or at the end of the fermentation step, wherein the low pH stable lactase retains its activity at a pH of 5.0 and a temperature of 37 °C at a level of at least 5 % as compared to its activity at the optimum pH of the lactase.

## Definitions

In connection with the present invention the following definitions apply:

"LAU" means "Lactose Units" and 1 lactase unit (1 LAU) is the amount of enzyme which releases 1 micromole glucose per minute in M-buffer at pH 6.5 and 37°C with a lactose concentration of 4.75% w/v. M-buffer is prepared by dissolving 3.98 g $C_6H_5Na_3O_7$-$2H_2O$, 8.31 g citric acid, 0.9 g $K_2SO_4$, 2.6 g $K_2HPO_4$, 7.35 g $KH_2PO_4$, 5.45 g KOH, 4.15 g $MgCl_2$-$6H_2O$, 3.75 g $CaCl_2$-$2H_2O$ and 1.4 g $NaHCO_3$ in 4 liter water, adding 12.5 ml 4N NaOH, adjusting to pH 6.5 using HCl, and adding water up to a total volume of 5 liter.

The activity in LAU of a specific lactase may be determined by direct measurement of glucose released from lactose under the conditions described above. The skilled person will know how to determine such activity. Alternatively, the activity may be determined by using the lactase activity assay described in Example 1 of the present application. Here, the activity is obtained by comparing to a standard curve run with a lactase of known activity, and the activity of the unknown sample calculated from this. The lactase of known activity may, e.g., be Lactozym obtained from Novozymes A/S, Denmark.

The expression "heat treatment" means any treatment using any temperature, for any period of time and by any means or equipment, which inactivates at least a portion of the bacteria of the starter culture. In this connection the term "inactivate" means any stop, reduction or inhibition of growth of the bacteria, e.g. cell lysing.

The expression "lactic acid bacteria" ("LAB") designates a gram-positive, microaerophilic or anaerobic bacteria, which ferment sugars with the production of acids including lactic acid as the predominantly produced acid, acetic acid and propionic acid. The industrially most useful lactic acid bacteria are found within the order "Lactobacillales" which includes *Lactococcus spp., Streptococcus spp., Lactobacillus spp., Leuconostoc spp.,* Pseudoleuconostoc spp., *Pediococcus spp., Brevibacterium spp., Enterococcus spp.* and *Propionibacterium spp.* These are frequently used as food cultures alone or in combination with other lactic acid bacteria.

Lactic acid bacteria, including bacteria of the species Lactobacillus sp. and Lactococcus sp., are normally supplied to the dairy industry either as frozen or freeze-dried cultures for bulk starter propagation or as so-called "Direct Vat Set" (DVS) cultures, intended for direct inoculation into a fermentation vessel or vat for the production of a dairy product, such as a fermented milk product or a cheese. Such lactic acid bacterial cultures are in general referred to as "starter cultures" or "starters". Typically, a starter culture for yogurt comprises *Streptococcus thermophilus* and *Lactobacillus delbrueckii* subsp. *bulgaricus,* and in most countries a yogurt is by legislation defined as a fermented milk product produced using a starter culture comprising the two said strains.

The term "milk" is to be understood as the lacteal secretion obtained by milking of any mammal, such as cows, sheep, goats, buffaloes or camels. In a preferred embodiment, the milk is cow's milk. The term milk also includes protein/fat solutions made of plant materials, e.g. soy milk.

The term "milk base" may be any raw and/or processed milk material that can be subjected to fermentation according to the method of the invention. Thus, useful milk bases include, but are not limited to, solutions/-suspensions of any milk or milk like products comprising protein, such as whole or low fat milk, skim milk, buttermilk, reconstituted milk powder, condensed milk, dried milk, whey, whey permeate, lactose, mother liquid from crystallization of lactose, whey protein concentrate, or cream. Obviously, the milk base may originate from any mammal, e.g. being substantially pure mammalian milk, or reconstituted milk powder.

Prior to fermentation, the milk base may be homogenized and pasteurized according to methods known in the art.

"Homogenizing" as used herein means intensive mixing to obtain a soluble suspension or emulsion. If homogenization is performed prior to fermentation, it may be performed so as to break up the milk fat into smaller sizes so that it no longer separates from the milk. This may be accomplished by forcing the milk at high pressure through small orifices.

"Pasteurizing" as used herein means treatment of the milk base to reduce or eliminate the presence of live organisms, such as microorganisms. Preferably, pasteurization is attained by maintaining a specified temperature for a specified period of time. The specified temperature is usually attained by heating. The temperature and duration may be selected in order to kill or inactivate certain bacteria, such as harmful bacteria. A rapid cooling step may follow.

"Fermentation" in the methods of the present invention means the conversion of carbohydrates into alcohols or acids through the action of a microorganism. Preferably, fermentation in the methods of the invention comprises conversion of lactose to lactic acid.

Fermentation processes to be used in production of dairy products are well known and the person of skill in the art will know how to select suitable process conditions, such as temperature, oxygen, amount and characteristics of microorganism(s) and process time. Obviously, fermentation conditions are selected so as to support the achievement of the present invention, i.e. to obtain a dairy product in solid (such as a cheese) or liquid form (such as a fermented milk product).

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The expression "fermented milk product" means a food or feed product wherein the preparation of the food or feed product involves fermentation of a milk base with a lactic acid bacteria. "Fermented milk product" as used herein includes but is not limited to products such as thermophilic fermented milk products, e.g. yoghurt, mesophilic fermented milk products, e.g. sour cream and buttermilk, as well as fermented whey.

The term "thermophile" herein refers to microorganisms that thrive best at temperatures above 35°C. The industrially most useful thermophilic bacteria include Streptococcus spp. and Lactobacillus spp. The term "thermophilic fermentation" herein refers to fermentation at a temperature above about 35°C, such as between about 35°C to about 45°C. The term "thermophilic fermented milk product" refers to fermented milk products prepared by thermophilic fermentation of a thermophilic starter culture and include such fermented milk products as set-yoghurt, stirred-yoghurt and drinking yoghurt, e.g. Yakult.

The term "mesophile" herein refers to microorganisms that thrive best at moderate temperatures (15°C-35°C). The industrially most useful mesophilic bacteria include Lactococcus spp. and Leuconostoc spp. The term "mesophilic fermentation" herein refers to fermentation at a temperature between about 22°C and about 35°C. The term "mesophilic fermented milk product" refers to fermented milk products prepared by mesophilic fermentation of a mesophilic starter culture and include such fermented milk products as buttermilk, sour milk, cultured milk, smetana, sour cream, Kefir and fresh cheese, such as quark, tvarog and cream cheese.

In connection with the present invention, "shear stress" may be measured by the following method:
Seven days after production, the fermented milk product was brought to 13°C and manually stirred gently by means of a spoon (5 times) until homogeneity of the sample. The rheological properties of the sample were assessed on a rheometer (Anton Paar Physica Rheometer with ASC, Automatic Sample Changer, Anton Paar® GmbH, Austria) by using a bob-cup. The rheometer was set to a constant temperature of 13 °C during the time of measurement. Settings were as follows:

Holding time (to rebuild to somewhat original structure)
5 minutes without any physical stress (oscillation or rotation) applied to the sample. Oscillation step (to measure the elastic and viscous modulus, G' and G", respectively, therefore calculating the complex modulus G*)

Constant strain = 0.3 %, frequency (f) = [0.5..8] Hz
6 measuring points over 60 s (one every 10 s)

Rotation step (to measure shear stress at 300 1/s)
Two steps were designed:

1) Shear rate = [0.3-300] 1/s and 2) Shear rate = [275-0.3] 1/s.

Each step contained 21 measuring points over 210 s (on every 10 s).
The shear stress at 300 1/s was chosen for further analysis, as this correlates to mouth thickness when swallowing a fermented milk product.

In connection with the present invention, "gel firmness" may be measured by the following method:
Gel firmness is measured by a back extrusion test with a texture analyzer (TA.XT Plus, Stable Micro System, Surrey, UK) supplied with a 35mm parallel plate. The travel distance is set to 15 mm, and the travel speed to 2 mm/s. The test is performed after 7 days from production. The fermented milk product was brought to 13°C and manually stirred gently, and measured in a 250 g plastic container. The maximal force (N or g) obtained by force versus distance curves is used as "gel firmness" parameter, the positive area (N* mm) as degree of deformation, the maximal negative force (N) as ropiness.

The term "low pH stable lactase" herein refers to a lactase, which retains its activity at a pH of 5.0 and a temperature of 37 °C at a level of at least 5 % as compared to its activity at the optimum pH of the lactase.

The term "activity at the optimum pH" means the lactase activity at the pH, where the lactase has its optimum activity.

The term "non-lactose carbohydrate" means any carbohydrate, which is not lactose, and which a lactose-deficient lactic acid bacterium used in the process of the invention is capable of metabolizing.

The expression "at the start of the fermentation step" means shortly before, at the same time as or shortly after addition of the starter culture to the milk base. Here, the term "shortly" means less than 30 minutes".

The expression "during the fermentation step" means at any time during the fermentation after the start and before the end of the fermentation.

The expression "at the end of the fermentation step" means shortly before, at the same time as or shortly after the target pH is reached. Here, the term "shortly" means less than 30 minutes".

The term "target pH" means the pH at which the fermentation step ends. Depending on various parameters of the process, the fermentation step is terminated by a method selected from the group consisting of 1) acidification of the fermented milk rendering at least one strain of the starter culture unable to grow, 2) cooling treatment and 3) depletion of the non-lactose carbohydrate.

## DEPOSITS AND EXPERT SOLUTION

The Applicant requests that a sample of the deposited microorganism should be made available only to an expert approved by the Applicant.

*Streptococcus thermophilus* strain deposited with DSMZ-Deutsche Sammlung von Mikroorganismen und Zellkulturen GmbH, Inhoffenstr. 7B, D-38124 Braunschweig, on 2014-06-12 under the accession no. DSM 28952.

*Streptococcus thermophilus* strain deposited with DSMZ-Deutsche Sammlung von Mikroorganismen und Zellkulturen GmbH, Inhoffenstr. 7B, D-38124 Braunschweig, on 2014-06-12 under the accession no. DSM 28953.

*Lactobacillus delbrueckii* ssp. *bulgaricus* strain deposited with DSMZ-Deutsche Sammlung von Mikroorganismen und Zellkulturen GmbH, Inhoffenstr. 7B, D-38124 Braunschweig, on 2014-06-12 under the accession no. DSM 28910;

The deposits were made according to the Budapest treaty on the international recognition of the deposit of microorganisms for the purposes of patent procedure.

## EXAMPLES

## Example 1

*Lactase activity-assay in Eppendorf tubes at 37oC, pH 6.5*

Principle:

Lactase hydrolyses lactose into glucose and galactose. Glucose is measured after a modified version of the common glucose oxidase / peroxidase assay (Werner, W. et al. (1970) Z. analyt. Chem. 252: 224.).

LAU is defined as the amount of enzyme liberating 1 micromole of glucose per min at 37°C, pH 6.5 in M-buffer (M-buffer is defined in the description of the present patent application). Alternatively, the activity in LAU for a specific lactase may be determined by the method described here. The value obtained is compared to a standard curve run with a lactase of known activity, and the activity of the unknown sample calculated from this. The lactase of known activity may, e.g., be Lactozym obtained from Novozymes A/S, Denmark.

Solutions:

Assay buffer: 50 mM succinate, 50 mM HEPES, 50 mM CHES, 150 mM KCl, 2 mM $CaCl_2$, 1 mM $MgCl_2$, 0.01% Triton X100, pH 6.5

GOD-Perid solution: 65 mM sodium phosphate, pH 7, 0.4 g/l Glucose oxidase, 0.013 g/l HRP (Horse Radish Peroxidase), 0.65 g/l ABTS (2,2'-azino-bis(3-ethylbenzthiazoline-6-sulphonic acid)).

Substrate:

160 mM lactose, 50 mM succinate, 50 mM HEPES, 50 mM CHES, 150 mM KCl, 2 mM $CaCl_2$, 1 mom $MgCl_2$, pH 6.5.

Standard:

Lactozym (available from Novozymes A/S, Denmark) with a known activity in LAU/g is used as standard, diluted in assay buffer in the range from 0.09 - 0.7 LAU/g.

Sam ples:

Enzyme samples are diluted appropriately in assay buffer.

Procedure:

1. 375 μl substrate is incubated 5 minutes at 37°C.
2. 25 μl enzyme diluted in assay buffer is added.
3. The reaction is stopped after 30 minutes by adding 60 μl 1 M NaOH
4. 20 μl is transferred to a 96 well microtiter plate and 200 μl GOD-Perid solution is added. After 30 minutes at room temperature, the absorbance is measured at 420 nm.

## Example 2

100 ml 15 or 30%(w/w) whey permeate containing primarily lactose and ions was made by mixing 15 or 30 g spray-dried whey permeate powder (Variolac, Aria) in 85 or 70 ml ionic water respectively. The solution was poured in a flask containing a magnetic stirring bar and placed in a water bath at 37°C. After 15 min, enzyme was added. Enzymes tested were Lactozym, a commercially available lactase from Novozymes A/S, Denmark, having an activity of 3060 LAU/g, and an experimental lactase from *Bifidobacterium bifidum* having the encoded sequence shown in SEQ ID NO: 2 and an activity of 295 LAU/g.

Dosages were 4225 LAU/l milk of Lactozym and 2025 LAU/l milk of the *Bifidobacterium* lactase. Milk samples were taken at regular intervals up till 5.5 hrs. and the enzyme was inactivated by heating to 99°C for 10 min in a thermomixer. Samples were diluted appropriately and filtered through a 0.20 μm filter.

Lactose hydrolysis was measured using a Dionex BioLC equipped with a Dionex PA1 column and a Pulsed Amperiometrisk Detektor (PAD). Peaks were identified and quantified by comparing with known standards of lactose, glucose and galactose.

Results are given below.

Table 1: Lactose, glucose and galactose in 15% DS whey permeate after treatment with Lactozym or *Bifidobacterium* lactase.

|  | Lactozym |  |  | *Bifidobacterium* lactase |  |  |
|---|---|---|---|---|---|---|
| Time min | Lactose mM | Glucose mM | Galactose mM | Lactose mM | Glucose mM | Galactose mM |
| 0 | 499 | 1 | 2 | 499 | 1 | 2 |
| 30 | 312 | 135 | 106 | 410 | 61 | 63 |
| 60 | 211 | 224 | 155 | 349 | 119 | 122 |
| 120 | 110 | 295 | 221 | 220 | 199 | 202 |
| 180 | 66 | 324 | 249 | 149 | 281 | 290 |
| 240 | 50 | 346 | 279 | 84 | 336 | 348 |
| 330 | 37 | 372 | 312 | 31 | 350 | 368 |

Table 2: Lactose, glucose and galactose in 30% DS whey permeate after treatment with Lactozym or *Bifidobacterium* lactase.

|  | Lactozym |  |  | *Bifidobacterium* lactase |  |  |
|---|---|---|---|---|---|---|
| Time min | Lactose mM | Glucose mM | Galactose mM | Lactose mM | Glucose mM | Galactose mM |
| 0 | 848 | 1 | 4 | 848 | 1 | 4 |
| 30 | 824 | 109 | 75 | 819 | 43 | 45 |
| 60 | 615 | 253 | 150 | 788 | 86 | 83 |
| 120 | 420 | 370 | 242 | 651 | 159 | 158 |
| 180 | 291 | 459 | 300 | 625 | 232 | 230 |
| 240 | 246 | 559 | 373 | 501 | 283 | 273 |
| 330 | 154 | 544 | 367 | 391 | 333 | 324 |
| 1440 | 54 | 649 | 545 | 20 | 727 | 739 |

Also when tested at higher lactose concentrations as in 15% or 30% whey permeate no or very little galactooligosaccharides are produced. Again, the produced galactose and glucose levels are equal and match the amount of lactose hydrolyzed. For comparison, Lactozym produces less galactose than glucose, clearly showing that galactooligosaccharides have been produced.

## Example 3

*pH profile (at 37°C) and temperature profile (at pH 6.5) of experimental lactase from Bifidobacterium bifidum using lactose as substrate.*

Principle:

Lactase hydrolyses lactose and glucose + galactose is formed. Glucose is measured after a modified version of the common glucose oxidase / peroxidase assay (Werner, W. et al. (1970) Z. analyt. Chem. 252: 224.)

*pH profile*

Substrate:

167 mM lactose, 50 mM succinate, 50 mM HEPES, 50 mM CHES, 150 mM KCl, 2 mM $CaCl_2$, 1 mM $MgCl_2$ and pH adjusted to pH 3, 4, 5, 6, 7, 8, 9 and 10 with NaOH.

Enzyme Sample:

Experimental lactase from *Bifidobacterium bifidum* having the encoded sequence shown in SEQ ID NO: 2 was diluted appropriately in 150 mM KCl, 2 mM $CaCl_2$, 1 mM $MgCl_2$, 0.01% Triton X100.

Procedure:

- 10 μl enzyme sample diluted in enzyme dilution buffer was added to PCR tubes at room temp.
- 90 μl substrate was added at room temp. and quickly placed in a Peltier Thermal Cycler (PCT-200, MJ research) at 37°C and incubated for 30 min and then placed on ice.
- The reaction was stopped by adding 100 μl 0.25 M NaOH.
- 20 μl was transferred to a 96 well microtitre plate and 230 μl 65 mM sodium phosphate, pH 7, 0.4 g/l Glucose oxidase, 0.013 g/l HRP, 0.65 g/l ABTS solution was added. After 30 minutes at room temperature, the absorbance was measured at 420 nm.

Table 3:

| pH | *B. bifidum* lactase relative activity (% of activity at pH6) |
|---|---|
| 3 | 0 |
| 4 | 4 |
| 5 | 75 |
| 6 | 100 |
| 7 | 85 |
| 8 | 39 |
| 9 | 10 |
| 10 | 4 |

*Temperature profile*

<u>Substrate:</u>

167 mM lactose, 50 mM succinate, 50 mM HEPES, 50 mM CHES, 150 mM KCl, 2 mM $CaCl_2$, 1 mM $MgCl_2$ and pH adjusted to pH 6.5 with NaOH.

<u>Enzyme Sample:</u>

Experimental lactase from *Bifidobacterium bifidum* having the encoded sequence shown in SEQ ID NO: 2 was diluted appropriately in 50 mM succinate, 50 mM HEPES, 50 mM CHES, 150 mM KCl, 2 mM $CaCl_2$, 1 mM $MgCl_2$, 0.01% Triton X100 and pH adjusted to pH 6.5.

<u>Procedure:</u>

- 10 $\mu$l enzyme sample diluted in enzyme dilution buffer was added to PCR tubes at room temp.
- 90 $\mu$l preheated (in a Peltier Thermal Cycler 30-70°C) substrate was added and incubation was performed with a temp. gradient from 30-70°C for 30 min. and then placed on ice.
- The reaction was stopped by adding 100 $\mu$l 0.25 M NaOH.
- 20 $\mu$l was transferred to a 96 well microtitre plate and 230 $\mu$l 65 mM sodium phosphate, pH 7, 0.4 g/l Glucose oxidase, 0.013 g/l HRP, 0.65 g/l ABTS solution was added. After 30 minutes at room temperature, the absorbance was measured at 420 nm.

Table 4:

| Temp. °C | *B. bifidum* lactase relative activity (% of activity at 38.1 °C) |
|---|---|
| 20 | 54 |
| 21 | 63 |
| 22 | 64 |
| 24 | 68 |
| 26 | 73 |
| 29 | 81 |
| 31 | 88 |
| 34 | 94 |
| 36 | 96 |
| 38 | 100 |
| 43 | 96 |
| 48 | 91 |
| 52 | 83 |
| 57 | 76 |
| 62 | 58 |
| 66 | 32 |
| 69 | 20 |
| 70 | 17 |

### Example 4

*Determination of Km for lactase enzymes at 5°C*

Principle:

Lactase hydrolyses lactose and glucose + galactose is formed. Glucose is measured after a modified version of the common glucose oxidase / peroxidase assay (Werner, W. et al. (1970) Z. analyt. Chem. 252: 224.)

Substrate:

Different lactose concentrations ranging from Km/5 to 10* Km, 50 mM succinate, 50 mM HEPES, 50 mM CHES, 150 mM KCl, 2 mM $CaCl_2$, 1 mM $MgCl_2$ and pH adjusted to pH 6.5 with NaOH.

Enzyme Sample:

Experimental lactase from *Bifidobacterium bifidum* having the encoded sequence shown in SEQ ID NO: 2 was diluted appropriately in 50 mM succinate, 50 mM HEPES, 50 mM CHES, 150 mM KCl, 2 mM $CaCl_2$, 1 mM $MgCl_2$, 0.01% Triton X100, pH adjusted to pH 6.5 with NaOH.

12 g/l Lactozym (commercially available lactase from Novozymes A/S, Denmark) was diluted 6000 times in the same buffer.

Procedure:

- 10 $\mu$l enzyme sample (5°C) was added to a 96 well microtitre plate on ice.
- 90 $\mu$l substrate (5°C) was added and incubated for 2 hours at 5°C.
- The reaction was stopped by adding 100 $\mu$l 0.25 M NaOH.
- 20 $\mu$l was transferred to a 96 well microtitre plate and 230 $\mu$l 65 mM sodium phosphate, pH 7, 0.4 g/l Glucose oxidase, 0.013 g/l HRP, 0.65 g/l ABTS solution was added. After 30 minutes at room temperature, the absorbance was measured at 420 nm.

Km determination:

Computerized nonlinear least-squares fitting and the Michaelis-Menten equation:

$$v = (Vmax * S)/(Km + S)$$

was used. Km for the *Bifidobacterium* lactase and Lactozym were determined to be 8 mM and 30 mM, respectively.
In a similar test performed at 37°C, Km for the *Bifidobacterium* lactase and Lactozym were determined to be 13 mM and 30 mM, respectively.

### Example 5

*Production of yogurt using lactase from Bifidobacterium bifidum and a lactose-deficient starter culture - Different levels of sucrose and lactase*

Experimental plan

For the selected combination of a low pH stable lactase and a lactose-deficient starter culture, two levels of added sucrose (0.5 % and 0.7 %), and two levels of added lactase (800 LAU/L and 1000 LAU/L), were tested. As reference fermentations with no added lactase and two levels of sucrose (1.0 % and 1.5 %) were carried out. The lactase was added at the start of the fermentation together with the starter culture.

Milk base

Table 5: Composition of milk base

|  | Amount (g) | Protein content (%) | Carbohydrate (%) | Fat (%) |
|---|---|---|---|---|
| Skim milk | 1000 | 3.5 | 4.8 | 0.1 |
| 1.5 % milk | 2000 | 3.4 | 4.7 | 1.5 |
| Protein powder "Promilk 802FB" | 14 | 80.0 |  |  |
| Protein powder "Milex 240" | 95 | 34.0 |  |  |
| Total milk base | 3109 | 4.71 | 4.57 | 1.00 |

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Measurements

Fat, protein and lactose levels were determined using MilkoScan analysis. Post-acidification was measured over a period of 28 days storage at 5 °C.

The level of sucrose, glucose, galactose, fructose and lactose in the fermented milk at day 1 and day 14 after production was measured by HPLC.

*Gel firmness*

Gel firmness is measured by a back extrusion test with a texture analyzer (TA.XT Plus, Stable Micro System, Surrey, UK) supplied with a 35mm parallel plate. The travel distance is set to 15 mm, and the travel speed to 2 mm/s. The test is performed after 7 days from production. The fermented milk product was brought to 13°C and manually stirred gently, and measured in a 250 g plastic container. The maximal force (g) obtained by force versus distance curves is used as "gel firmness" parameter.

*Shear stress*

Seven days after incubation, the fermented milk product was brought to 13°C and manually stirred gently by means of a spoon (5 times) until homogeneity of the sample. The rheological properties of the sample were assessed on a rheometer (Anton Paar Physica Rheometer with ASC, Automatic Sample Changer, Anton Paar® GmbH, Austria) by using a bob-cup. The rheometer was set to a constant temperature of 13 °C during the time of measurement. Settings were as follows:

Holding time (to rebuild to somewhat original structure)
5 minutes without any physical stress (oscillation or rotation) applied to the sample. Oscillation step (to measure the elastic and viscous modulus, G' and G", respectively, therefore calculating the complex modulus G*)

Constant strain = 0.3 %, frequency (f) = [0.5..8] Hz
6 measuring points over 60 s (one every 10 s)

Rotation step (to measure shear stress at 300 1/s)
Two steps were designed:

1) Shear rate = [0.3-300] 1/s and 2) Shear rate = [275-0.3] 1/s.

Each step contained 21 measuring points over 210 s (on every 10 s).
The shear stress at 300 1/s was chosen for further analysis, as this correlates to mouth thickness when swallowing a fermented milk product.

Procedure

The ingredients of the milk base were mixed and allowed to re-hydrate for 2 hours at 5 °C. The milk base was then pasteurized at 90 °C for 20 minutes. The fermentation was carried out at 43 °C to an end pH of 4.55 to form yogurt. The yogurt was cooled in a PTU (Post Treatment Unit) at a cooling temperature of 25 °C at 2 bars. The cooled yogurt was stored at 6 °C.

Results

*Post-acidification*

Table 6: Post-acidification

|  | Ref. 1 | Ref. 2 | Test Sample 1 | Test Sample 2 | Test Sample 3 | Test Sample 4 |
|---|---|---|---|---|---|---|
| Sucrose (%) | 1 | 1.5 | 0.5 | 0.5 | 0.7 | 0.7 |
| Lactase ( LAU/ L) | 0 | 0 | 800 | 1000 | 800 | 1000 |
| Ferment. time (Hours: Min.) | 7:25 | 7:05 | 7:15 | 7:15 | 7:00 | 7:00 |
| End pH | 4.58 | 4.55 | 4.55 | 4.55 | 4.55 | 4.55 |
| pH Day 1 | 4.58 | 4.53 | 4.54 | 4.53 | 4.50 | 4.51 |
| pH Day 7 | 4.54 | 4.49 | 4.50 | 4.50 | 4.47 | 4.49 |
| pH Day 14 | 4.49 | 4.43 | 4.49 | 4.49 | 4.46 | 4.47 |
| pH Day 21 | 4.35 | 4.34 | 4.45 | 4.45 | 4.42 | 4.43 |
| pH Day 28 | 4.35 | 4.34 | 4.45 | 4.42 | 4.43 | 4.42 |
| pH drop | 0.23 | 0.19 | 0.09 | 0.11 | 0.07 | 0.09 |

For all six samples, i.e. 2 reference samples and 4 samples produced according to the invention (test samples), a pH of about 4.55 was reached in 7 hours.
As will appear from Table 6, the post-acidification over a period of 28 days was strongly reduced for the samples produced according to the invention as compared to the reference samples.

*Carbohydrate analysis*

Table 7: Residual carbohydrate levels after 1 day from production

|  | Ref. 1 | Ref. 2 | Test Sample 1 | Test Sample 2 | Test Sample 3 | Test Sample 4 |
|---|---|---|---|---|---|---|
| Sucrose (%) start ferment. | 1 | 1.5 | 0.5 | 0.5 | 0.7 | 0.7 |
| Lactase ( LAU/ L) | 0 | 0 | 800 | 1000 | 800 | 1000 |
| Fructose (mg/g) | 1.62 | 1.91 | 0.80* | 0.74* | 1.27 | 1.18 |
| Galactose (mg/g) | < 0.9 | 1.78* | 29.01 | 29.87 | 28.78 | 29.29 |

(continued)

|  | Ref. 1 | Ref. 2 | Test Sample 1 | Test Sample 2 | Test Sample 3 | Test Sample 4 |
|---|---|---|---|---|---|---|
| Glucose (mg/g) | 0.77* | 1.73 | 27.60 | 27.84 | 28.08 | 28.19 |
| Lactose (mg/g) | 54.16 | 53.37 | 1.18 | 0.40 | 1.20 | 0.40 |
| Sucrose (mg/g) | < 0.3 | 4.6 | < 0.3 | < 0.3 | 1.3 | 0.7 |
| *value is between Limit of Detection and Limit of Quantification. | | | | | | |

All levels of Table 7 are mean values of two samples.

For the 4 test samples, at the end of fermentation the level of lactose was very low and the level of glucose and galactose was very high as compared to the reference samples indicating high activity of the added lactase. For the test samples using a lactase level of 1000 LAU/L a residual lactose level of approx. 0.04 % was obtained, and for the test samples using a lactase level of 800 LAU/L a residual lactose level of approx. 0.1 % was obtained.

Fermented milk having a low lactose level and a high level of glucose and galactose has a much higher level of perceived sweetness than fermented milk with a high lactose level and a low level of glucose and galactose like the reference samples. The reason for this is that glucose and galactose has a much higher sweetness index than lactose.

*Gel firmness*

Table 8: Gel firmness

|  | Ref. 1 | Ref. 2 | Test Sample 1 | Test Sample 2 | Test Sample 3 | Test Sample 4 |
|---|---|---|---|---|---|---|
| Sucrose (%) start ferment. | 1 | 1.5 | 0.5 | 0.5 | 0.7 | 0.7 |
| Lactase ( LAU/ L) | 0 | 0 | 800 | 1000 | 800 | 1000 |
| Gel firmness (g) | 45.76 | 47.42 | 48.19 | 54.57 | 50.19 | 46.60 |

All levels of Table 8 are mean values of two samples.

As will appear from Table 8, 3 out of the 4 test samples had significantly higher gel firmness than the 2 reference samples.

*Shear stress*

Table 9: Shear stress measured at 300 $s^{-1}$

|  | Ref. 1 | Ref. 2 | Test Sample 1 | Test Sample 2 | Test Sample 3 | Test Sample 4 |
|---|---|---|---|---|---|---|
| Sucrose (%) start ferment. | 1 | 1.5 | 0.5 | 0.5 | 0.7 | 0.7 |
| Lactase ( LAU/ L) | 0 | 0 | 800 | 1000 | 800 | 1000 |
| Shear stress (Pa) | 67.1 | 65.3 | 78.2 | 84.4 | 84.7 | 77.6 |

All levels of Table 9 are mean values of two samples.

As will appear from Table 9, the 4 test samples had significantly higher shear stress measured at 300 $s^{-1}$ than the 2 reference samples. In the group of 4 test samples, the combinations of 1) a level of added sucrose of 0.5 % and a lactase level of 1000 LAU/L, and 2) a level of added sucrose of 0.7 % and a lactase level of 800 LAU/L, had the highest shear stress.

**Example 6**

*Production of yogurt using lactase from Bifidobacterium bifidum and a lactose-deficient starter culture - addition of excess level of sucrose before fermentation I*

Experimental plan

Fermentations were carried out with a reference sample containing no lactase and 9.5 % sucrose and 2 test samples containing 800 LAU/L of lactase and 6.5 % and 7.0 % sucrose. The lactase was added at the start of the fermentation together with the starter culture.

Milk base

Table 10: Composition of basic milk base 1

|  | Amount (g) | Protein content (%) | Carbohydrate (%) | Fat (%) |
|---|---|---|---|---|
| Skim milk | 984 | 3.5 | 4.8 | 0.1 |
| 1.5 % milk | 1911 | 3.4 | 4.7 | 1.5 |
| Protein powder "Promilk 802FB" | 13.3 | 80.0 |  |  |
| Protein powder "Milex 240" | 91.5 | 34.0 | 54.00 | 1.00 |
| Total milk base | 3000 | 4.71 | 4.57 | 1.02 |

Table 11: Composition of basic milk base 2

|  | Amount (g) | Protein content (%) | Carbohydrate (%) | Fat (%) |
|---|---|---|---|---|
| Skim milk | 984 | 3.5 | 4.8 | 0.1 |
| 1.5 % milk | 1911 | 3.4 | 4.7 | 1.5 |
| Protein powder "Promilk 802FB" | 17.40 | 80.0 |  |  |
| Protein powder "Milex 240" | 109.50 | 34.0 | 5400 | 1.00 |
| Sucrose | 180.00 |  | 100.0 |  |
| Total milk base | 3202 | 4.70 | 11.75 | 0.96 |

Basic milk base 2 was used to prepare the final milk bases for the reference sample and the two test samples by addition of additional sucrose.

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Measurements

All measurements were carried out using the same methods as in Example 5.

Procedure

The ingredients of the milk base were mixed and allowed to re-hydrate for 2 hours at 5 °C. The milk base was then pasteurized at 95 °C for 5 minutes. The milk base was then homogenized at 65 °C at 200 bar. The fermentation was carried out at 43 °C to an end pH of 4.55 to form yogurt. The yogurt was cooled in a PTU (Post Treatment Unit) at a cooling temperature of 25 °C at 2 bars. The cooled yogurt was stored at 6 °C.

Results

*Post-acidification*

Table 12: Post-acidification

| Sample | 1 | 2 | 3 |
|---|---|---|---|
| Lactase (LAU/L) | 0 | 800 | 800 |
| Fermentation time | 6 h 15 min | 6 h 15 min | 6 h 15 min |
| End pH | 4.55 | 4.51 | 4.51 |
| pH Day 4 | 4.54 | 4.44 | 4.44 |
| pH Day 7 | 4.51 | 4.42 | 4.42 |
| pH Day 14 | 4.40 | 4.38 | 4.37 |
| pH Day 21 | 4.38 | 4.38 | 4.37 |
| pH Day 28 | 4.36 | 4.37 | 4.36 |
| pH drop | 0.19 | 0.14 | 0.15 |

*Carbohydrate analysis*

Table 13: Carbohydrate analysis

| Sample | 1 | | 2 | | 3 | |
|---|---|---|---|---|---|---|
| Lactase (LAU/L) | 0 | | 800 | | 800 | |
| Sucrose (%) start ferment. | 9.5 | | 6.5 | | 7.0 | |
| | Day 1 | Day 28 | Day 1 | Day 28 | Day 1 | Day 28 |
| Fructose (mg/g) | 2.4 | 3.07 | 1.7 | 2.01 | 1.9 | 2.00 |
| Galactose (mg/g) | < 2.0 | 8.99 | 26.4 | 29.03 | 26.4 | 28.72 |
| Glucose (mg/g) | < 1.0 | 8.84 | 26.2 | 27.52 | 26.2 | 27.22 |
| Lactose (mg/g) | 51.4 | 38.04 | 2.8 | < 2 | 2.7 | < 2 |
| Sucrose (mg/g | 64.3 | 64.23 | 41.7 | 42.38 | 46.0 | 46.26 |

All levels of Table 13 are mean values of two samples.
As will appear from Table 13, for the 2 test samples, at the end of fermentation the level of lactose was very low and the level of glucose and galactose was very high as compared to the reference sample indicating high activity of the added lactase.
Fermented milk having a low lactose level and a high level of glucose and galactose has a much higher level of sweetness than fermented milk with a high lactose level and a low level of glucose and galactose like the reference samples. The reason for this is that glucose and galactose has a much higher sweetness than lactose.

*Gel firmness and shear stress*

Table 14: Gel firmness and shear stress at 300 s$^{-1}$

| Sample | 1 | | 2 | | 3 | |
|---|---|---|---|---|---|---|
| Lactase (LAU/L) | 0 | | 800 | | 800 | |
| Sucrose (%) start ferment. | 9.5 | | 6.5 | | 7.0 | |
| Gel firmness (g) | 46.36 | | 46.76 | | 45.75 | |
| Shear stress at 1 s-1 (Pa) | 9.4 | 9.2 | 9.3 | 8.8 | 10.3 | 10.2 |
| Shear stress at 30.2 s-1 (Pa) | 39.2 | 38.8 | 41.6 | 39.2 | 44.7 | 45.1 |
| Shear stress at 135 s-1 (Pa) | 58.3 | 58.0 | 67.2 | 64.5 | 72.3 | 72.2 |
| Shear stress at 300 s-1 (Pa) | 69.4 | 69.1 | 80.6 | 78.2 | 84.4 | 84.4 |

As will appear from Table 14, the shear stress at 300 s$^{-1}$ of the two test samples containing lactase was significantly increased as compared to the reference sample with no lactase.

## Example 7

*Production of yogurt using lactase from Bifidobacterium bifidum and a lactose-deficient starter culture - addition of excess level of sucrose before fermentation II*

Experimental plan

Fermentations were carried out with two reference samples containing no lactase and 1.5 % and 9.5 % sucrose and 3 test samples containing 1000 LAU/L of lactase and 0.5 %, 6.5 % and 7.0 % sucrose. The lactase was added at the start of the fermentation together with the starter culture.

Milk base

The two basic milk bases of Example 6 were used to prepare the final milk bases for the reference samples and the three test samples by addition of additional sucrose.

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Measurements

All measurements were carried out using the same methods as in Example 5.

Procedure

The ingredients of the milk base were mixed and allowed to re-hydrate for 2 hours at 5 °C. The milk base was then pasteurized at 90 °C for 20 minutes. The fermentation was carried out at 43 °C to an end pH of 4.55 to form yogurt. The yogurt was cooled in a PTU (Post Treatment Unit) at a cooling temperature of 25 °C at 2 bars. The cooled yogurt was stored at 6 °C.

Results

*Post-acidification*

Table 15: Post-acidification

| Sam ple | 1 | 2 | 3 | 4 | 5 |
|---|---|---|---|---|---|
| Lactase (LAU/L) | 0 | 1000 | 0 | 1000 | 1000 |
| Fermentation time | 6 h 50 min | 6 h 55 min | 6 h 35 min | 6 h 10 min | 5 h 40 min |
| End pH | 4.55* | 4.55* | 4.55* | 4.55* | 4.55* |
| pH Day 1 | 4.54 | 4.51 | 4.52 | 4.48 | 4.47 |
| pH Day 7 | 4.45 | 4.45 | 4.40 | 4.34 | 4.35 |
| pH Day 14 | 4.37 | 4.44 | 4.37 | 4.32 | 4.34 |
| pH Day 21 | 4.35 | 4.39 | 4.35 | 4.29 | 4.29 |
| pH Day 28 | 4.34 | 4.37 | 4.33 | 4.27 | 4.26 |
| pH drop | 0.21 | 0.18 | 0.22 | 0.28 | 0.29 |
| *The fermentation is stopped by cooling at pH = 4.55. | | | | | |

As will appear from Table 15, the post-acidification was at the same level for the reference samples containing no lactase and the test samples containing lactase.

*Carbohydrate analysis*

Table 16: Carbohydrate analysis Day 1

| Sam ple | 1 | 2 | 3 | 4 | 5 |
|---|---|---|---|---|---|
| Lactase ( LAU/ L) | 0 | 1000 | 0 | 1000 | 1000 |
| Sucrose (%) start ferment. | 1.5 | 0.5 | 9.5 | 6.5 | 7.0 |
| Fructose (mg/g) | 1.96 | < 1 | 2.70 | 1.74 | 1.88 |
| Galactose (mg/g) | < 2 | 29.19 | < 2 | 27.83 | 27.89 |
| Glucose (mg/g) | < 1 | 27.37 | < 1 | 27.29 | 27.40 |
| Lactose (mg/g) | 56.11 | < 2 | 54.56 | < 2 | < 2 |
| Sucrose (mg/g | 5.45 | < 4 | 63.44 | 39.79 | 45.44 |

Table 17: Carbohydrate analysis Day 28

| Sam ple | 1 | 2 | 3 | 4 | 5 |
|---|---|---|---|---|---|
| Lactase ( LAU/ L) | 0 | 1000 | 0 | 1000 | 1000 |
| Sucrose (%) start ferment. | 1.5 | 0.5 | 9.5 | 6.5 | 7.0 |
| Fructose (mg/g) | 1.88 | < 1 | 4.72 | 2.95 | 3.39 |
| Galactose (mg/g) | 3.90 | 28.83 | < 2 | 28.79 | 29.15 |
| Glucose (mg/g) | 2.69 | 26.78 | 1.66 | 26.83 | 27.27 |
| Lactose (mg/g) | 50.71 | < 2 | 52.42 | < 2 | < 2 |

(continued)

| Sam ple | 1 | 2 | 3 | 4 | 5 |
|---|---|---|---|---|---|
| Sucrose (mg/g | 4.18 | < 4 | 62.07 | 39.91 | 46.17 |

As will appear from Tables 16 and 17, for the 3 test samples, at the end of fermentation the level of lactose was very low and the level of glucose and galactose was very high as compared to the reference sample indicating high activity of the added lactase.

Fermented milk having a low lactose level and a high level of glucose and galactose has a much higher level of sweetness than fermented milk with a high lactose level and a low level of glucose and galactose like the reference samples. The reason for this is that glucose and galactose has a much higher sweetness than lactose.

*Gel firmness and shear stress*

Table 18: Gel firmness and shear stress

| Sam ple | 1 | | 2 | | 3 | | 4 | | 5 | |
|---|---|---|---|---|---|---|---|---|---|---|
| Lactase ( LAU/L) | 0 | | 1000 | | 0 | | 1000 | | 1000 | |
| Sucrose (%) start ferment. | 1.5 | | 0.5 | | 9.5 | | 6.5 | | 7.0 | |
| Gel firmness (g) | 51.13 | | 49.41 | | 45.04 | | 45.94 | | 47.89 | |
| Shear stress at 1 s-1 (Pa) | 9.4 | 9.3 | 8.4 | 8.5 | 8.1 | 8.5 | 7.7 | 7.8 | 8.3 | 8.5 |
| Shear stress at 30.2 s-1 (Pa) | 39.4 | 38.5 | 38.2 | 37.6 | 34.3 | 34.7 | 37.3 | 37.1 | 38.8 | 38.9 |
| Shear stress at 135 s-1 (Pa) | 61.0 | 59.5 | 65.8 | 65.1 | 56.8 | 56.8 | 72.7 | 72.3 | 74.8 | 74.9 |
| Shear stress at 300 s-1 (Pa) | 72.1 | 70.5 | 78.8 | 77.6 | 69.1 | 69.1 | 86.7 | 86.6 | 89.4 | 89.4 |

As will appear from Table 18, the shear stress of the three test samples containing lactase was significantly increased as compared to the reference sample with no lactase.

**Example** 8

*Production of yogurt using lactase from Bifidobacterium bifidum and a lactose-deficient starter culture - addition of lactase before and after fermentation*

Experimental plan

Table 19: Experimental plan

| Sample | Timing of lactase addition | Milk Base | Sucrose (%) | Lactase (LAU/L) |
|---|---|---|---|---|
| 1 | None | 1 | 0.70 | 0 |
| 2 | Together with culture | 1 | 0.70 | 600 |
| 3 | Together with culture | 1 | 0.70 | 800 |
| 4 | Together with culture | 1 | 0.70 | 1000 |
| 5 | Together with culture | 1 | 0.70 | 1400 |
| 6 | None | 2 | 0.97 | 0 |
| 7 | After fermentation | 2 | 0.97 | 800 |
| 8 | After fermentation | 2 | 0.97 | 1600 |

(continued)

| Sample | Timing of lactase addition | Milk Base | Sucrose (%) | Lactase (LAU/L) |
|--------|---------------------------|-----------|-------------|-----------------|
| 9 | After fermentation | 2 | 0.97 | 2400 |
| 10 | After fermentation | 2 | 0.97 | 3200 |

The fermentation temperature was 43 °C. The end pH was 4.50. The ingredients of the milk base were mixed and allowed to re-hydrate for 2 hours at 6 °C. The milk base was then pasteurized at 95 °C for 5 minutes and homogenized at 200/50 bar at 65 °C. The fermentation was carried out at 43 °C to an end pH of 4.50 to form yogurt. The yogurt was cooled to 6 °C. The cooled yogurt was stored at 6 °C.

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Milk base

Table 20: Composition of milk base 1

|  | Amount (g) | Protein content (%) | Carbohydrate (%) | Fat (%) |
|--|-----------|---------------------|------------------|---------|
| Sucrose | 22 | 0.0 | 100.0 | 0.0 |
| 0.5 % milk | 1500 | 3.8 | 4.8 | 0.5 |
| 1.5 % milk | 1500 | 3.6 | 4.7 | 1.5 |
| Arla Skimmed Milk Powder | 103 | 34.0 | 54.0 | 1.0 |
| Total milk base | 3125 | 4.67 | 7.05 | 0.98 |

Table 21: Composition of milk base 2

|  | Amount (g) | Protein content (%) | Carbohydrate (%) | Fat (%) |
|--|-----------|---------------------|------------------|---------|
| Sucrose | 30 | 0.0 | 100.0 | 0.0 |
| 0.5 % milk | 1500 | 3.8 | 4.8 | 0.5 |
| 1.5 % milk | 1500 | 3.6 | 4.7 | 1.5 |
| Arla Skimmed Milk Powder | 103 | 34.0 | 54.0 | 1.0 |
| Total milk base | 3133 | 4.66 | 7.30 | 1.00 |

Measurem ents

All measurements were carried out using the same methods as in Example 5.

Results

*Post-acidification*

Table 22: Post-acidification

| Sample | Timing of lactase addition | Milk Base | Lactase (LAU/L) | pH Day 0 | pH Day 7 | pH Day 28 | pH Day 42 | pH drop |
|---|---|---|---|---|---|---|---|---|
| 1 | None | 1 | 0 | 4.89* | 4.89* | 4.87 | 4.84 | 0.05 |
| 2 | Together with culture | 1 | 600 | 4.26 | 4.23 | 4.23 | 4.22 | 0.04 |
| 3 | Together with culture | 1 | 800 | 4.22 | 4.26 | 4.24 | 4.24 | -0.02 |
| 4 | Together with culture | 1 | 1000 | 4.32 | 4.26 | 4.26 | 4.26 | 0.06 |
| 5 | Together with culture | 1 | 1400 | 4.36 | 4.30 | 4.28 | 4.27 | 0.09 |
| 6 | None | 2 | 0 | 4.47 | 4.47 | 4.47 | 4.44 | 0.03 |
| 7 | After fermentation | 2 | 800 | 4.43 | 4.40 | 4,42 | 4.38 | 0.05 |
| 8 | After fermentation | 2 | 1600 | 4.44 | 4.40 | 4,42 | 4.39 | 0.05 |
| 9 | After fermentation | 2 | 2400 | 4.41 | 4.43 | 4.40 | 4.40 | 0.01 |
| 10 | After fermentation | 2 | 3200 | NA | 4.43 | 4.41 | 4.40 | NA |
| * Insufficient sucrose to obtain fermentation to target pH. Therefore, this sample cannot be used as reference. | | | | | | | | |

As will appear from Table 22, the post-acidification over a period of 42 days was at a low level of below 0.09 for all lactase levels tested with lactase addition at the start of fermentation and at an even lower level of below 0.05 for all lactase levels tested with lactase addition at the end of fermentation. The addition of lactase at the end of the fermentation did not result in a statistically different post-acidification as compared to the reference sample with no added lactase. Thus, the present experiment shows that the use of a low pH stable lactase in combination with a lactose-deficient starter culture does not result in an unacceptable increase of the low post-acidification, which may be obtained with such a starter culture. This is true both when the lactase is added at the start and at the end of fermentation.

*Gel firmness and shear stress*

Table 23: Gel firmness and Shear Stress

| Sample | Timing of lactase addition | Milk Base | Sucrose (%) | Lactase (LAU/L) | Gel firmness (g) | Shear Stress (Pa) |
|---|---|---|---|---|---|---|
| 1 | None | 1 | 0.70 | 0 | 0.187* | 46.4* |
| 2 | Together with culture | 1 | 0.70 | 600 | 0.281 | 63.9 |
| 3 | Together with culture | 1 | 0.70 | 800 | 0.302 | 65.3 |
| 4 | Together with culture | 1 | 0.70 | 1000 | 0.318 | 64.5 |
| 5 | Together with culture | 1 | 0.70 | 1400 | 0.307 | 63.7 |
| 6 | None | 2 | 0.97 | 0 | 0.304 | 53.1 |
| 7 | After fermentation | 2 | 0.97 | 800 | 0.312 | 51.4 |
| 8 | After fermentation | 2 | 0.97 | 1600 | 0.322 | 52.8 |
| 9 | After fermentation | 2 | 0.97 | 2400 | 0.323 | 52.9 |

(continued)

| Sample | Timing of lactase addition | Milk Base | Sucrose (%) | Lactase (LAU/L) | Gel firmness (g) | Shear Stress (Pa) |
|---|---|---|---|---|---|---|
| 10 | After fermentation | 2 | 0.97 | 3200 | 0.320 | 51.7 |
| * Insufficient sucrose to obtain fermentation to target pH. Therefore, this sample cannot be used as reference. | | | | | | |

As will appear from Table 23, high levels of both gel firmness and shear stress was obtained for all lactase levels tested with lactase addition at the start of fermentation. For lactase levels tested with lactase addition at the end of fermentation, the levels of gel firmness and shear stress are somewhat lower, which is due to the mixing of the lactase into the yogurt, which partly disrupts the texture of the yogurt. For all lactase levels tested with lactase addition at the end of fermentation, the gel firmness is slightly higher than the reference sample with no added lactase. For all lactase levels tested with lactase addition at the end of fermentation, the shear stress is maintained at the same level or slightly lower.

*Carbohydrate analysis*

Table 24: Carbohydrate analysis

| Sam-ple | Lactase (LAU/L) | Galactose Day 20 (mg/g) | Glucose Day 20 (mg/g) | Sucrose Day 20 (mg/g) | Lactose Day 20 (mg/g) | Lactose 24 hours (%) | Lactose Day 20 (%) |
|---|---|---|---|---|---|---|---|
| 1 | 0 | 2.4 | 2.3 | < 1.0 | 50.7 | ND | ND |
| 2 | 600 | 29.5 | 27.7 | < 1.0 | < 0.5 | > 0.1 | 0.023 |
| 3 | 800 | 30.2 | 28.1 | < 1.0 | < 0.5 | 0.028 | 0.016 |
| 4 | 1000 | 30.0 | 27.8 | < 1.0 | < 0.5 | 0.014 | 0.014 |
| 5 | 1400 | 29.5 | 27.4 | < 1.0 | < 0.5 | 0.006 | 0.013 |
| 6 | 0 | 4.2 | 4.2 | < 1.0 | 49.2 | ND | ND |
| 7 | 800 | 31.2 | 30.7 | < 1.0 | < 0.5 | > 0.2 | 0.014 |
| 8 | 1600 | 30.7 | 30.2 | < 1.0 | < 0.5 | > 0.1 | 0.012 |
| 9 | 2400 | 31.2 | 30.6 | < 1.0 | < 0.5 | 0.029 | 0.011 |
| 10 | 3200 | 31.8 | 31.2 | < 1.0 | < 0.5 | 0.015 | 0.011 |

As will appear from Table 24, at Day 20 high concentrations of galactose and glucose are formed for all lactase levels tested both with lactase addition at the start and at the end of fermentation. Also, at Day 20 the level of glucose was below the detection limit of 0.5 mg/g, which qualifies as lactose free in some countries. For the higher levels of lactase, most of the lactose removal has been obtained 24 hours after the end of fermentation.

## Example 9

*Production of yogurt using lactase from Bifidobacterium bifidum and a lactose-deficient starter culture - addition of lactase before fermentation II*

Experimental plan

Table 25: Experimental plan

| Sample | Sucrose (%) | Lactase (LAU/L) |
|---|---|---|
| 1 | 0 | 0 |
| 2 | 0 | 1000 |
| 3 | 0 | 1200 |
| 4 | 0 | 1600 |
| 5 | 0.2 | 0 |
| 6 | 0.2 | 1000 |
| 7 | 0.2 | 1200 |
| 8 | 0.2 | 1600 |
| 9 | 0.7 | 0 |
| 10 | 0.7 | 1000 |
| 11 | 0.7 | 1200 |
| 12 | 0.7 | 1600 |

The fermentation temperature was 43 °C. The end pH was 4.45. The ingredients of the milk base were mixed and allowed to re-hydrate for 2 hours at 6 °C. The milk base was then pasteurized at 95 °C for 5 minutes and homogenized at 200/50 bar at 65 °C. The fermentation was carried out at 43 °C to an end pH of 4.45 to form yogurt. The yogurt was cooled to 5 °C. The cooled yogurt was stored at 6 °C.

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Milk base

Table 26: Composition of milk base

| | Amount (g) | Protein content (%) | Carbohydrate (%) | Fat (%) |
|---|---|---|---|---|
| 0.5 % milk | 1500 | 3.8 | 4.8 | 0.5 |
| 1.5 % milk | 1500 | 3.6 | 4.7 | 1.5 |
| Arla Skimmed Milk Powder | 103 | 34.0 | 54.0 | 1.0 |
| Total milk base | 3103 | 4.70 | 6.39 | 0.99 |

Measurements

All measurements were carried out using the same methods as in Example 5.

Results

*Post-acidification*

Table 27: Post-acidification

| Sample | Sucrose (%) | Lactase (LAU/L) | pH Day 0 | pH Day 1 | pH Day 14 | pH Day 28 | pH Day 42 | pH drop |
|--------|-------------|-----------------|----------|----------|-----------|-----------|-----------|---------|
| 1 | 0 | 0 | ND | ND | ND | ND | ND | ND |
| 2 | 0 | 1000 | 4.45 | 4.39 | 4.36 | 4.33 | 4.39 | 0.06 |
| 3 | 0 | 1200 | 4.45 | 4.39 | 4.38 | 4.34 | 4.39 | 0.06 |
| 4 | 0 | 1600 | 4.46 | 4.39 | 4.38 | 4.34 | 4.39 | 0.07 |
| 5 | 0.2 | 0 | ND | ND | ND | ND | ND | ND |
| 6 | 0.2 | 1000 | 4.50 | 4.44 | 4.40 | 4.40 | 4.43 | 0.07 |
| 7 | 0.2 | 1200 | 4.53 | 4.47 | 4.46 | 4.42 | 4.46 | 0.07 |
| 8 | 0.2 | 1600 | 4.53 | 4.49 | 4.46 | 4.44 | 4.47 | 0.06 |
| 9 | 0.7 | 0 | 4.76 | 4.73 | 4.69 | 4.68 | 4.70 | 0.06 |
| 10 | 0.7 | 1000 | 4.44 | 4.40 | 4.39 | 4.33 | 4.38 | 0.06 |
| 11 | 0.7 | 1200 | 4.46 | 4.40 | 4.39 | 4.33 | 4.39 | 0.07 |
| 12 | 0.7 | 1600 | 4.47 | 4.41 | 4.40 | 4.34 | 4.41 | 0.06 |

As will appear from Table 27, the post-acidification over a period of 42 days was at a low very level of approx. 0.06 for all lactase and sucrose levels tested. Thus, the present experiment shows that the use of a low pH stable lactase in combination with a lactose-deficient starter culture does not result in an unacceptable increase of the low post-acidification, which may be obtained with such a starter culture.

Gel *firmness and shear stress*

Table 28: Gel firmness and Shear Stress

| Sample | Sucrose (%) | Lactase (LAU/L) | Gel firmness (g) | Shear Stress (Pa) |
|--------|-------------|-----------------|------------------|-------------------|
| 1 | 0 | 0 | ND | ND |
| 2 | 0 | 1000 | 0.386 | 70.3 |
| 3 | 0 | 1200 | 0.377 | 69.8 |
| 4 | 0 | 1600 | 0.388 | 69.5 |
| 5 | 0.20 | 0 | ND | ND |
| 6 | 0.20 | 1000 | 0.380 | 70.7 |
| 7 | 0.20 | 1200 | 0.378 | 69.5 |
| 8 | 0.20 | 1600 | 0.397 | 70.2 |
| 9 | 0.70 | 0 | ND | ND |
| 10 | 0.70 | 1000 | 0.362 | 71.2 |
| 11 | 0.70 | 1200 | 0.375 | 72.2 |
| 12 | 0.70 | 1600 | 0.362 | 71.9 |

As will appear from Table 28, high levels of both gel firmness and shear stress was obtained for all lactase levels tested. The gel firmness and shear strength are at the same order of magnitude for all three levels of lactase tested.

*Carbohydrate analysis*

Table 29: Carbohydrate analysis

| Sam -ple | Sucrose (%) | Lactase (LAU/L) | Fructose (mg/g) | Galactose (mg/g) | Glucose (mg/g) | Lactose (mg/g) | Sucrose (mg/g) |
|---|---|---|---|---|---|---|---|
| 1 | 0 | 0 | < 0.5 | 6.3 | < 0.5 | 45.5 | <2 |
| 2 | 0 | 1000 | < 0.5 | 30.7 | 26.0 | < 0.9 | <2 |
| 3 | 0 | 1200 | < 0.5 | 31.5 | 26.6 | < 0.9 | <2 |
| 4 | 0 | 1600 | < 0.5 | 30.9 | 25.8 | < 0.9 | <2 |
| 5 | 0.20 | 0 | < 0.5 | 11.7 | 6.7 | 36.1 | <2 |
| 6 | 0.20 | 1000 | < 0.5 | 30.8 | 27.8 | < 0.9 | <2 |
| 7 | 0.20 | 1200 | < 0.5 | 31.6 | 28.3 | < 0.9 | <2 |
| 8 | 0.20 | 1600 | < 0.5 | 31.5 | 28.0 | < 0.9 | <2 |
| 9 | 0.70 | 0 | 1.1 | 9.1 | 9.1 | 43.4 | <2 |
| 10 | 0.70 | 1000 | 1,1 | 31.1 | 29.5 | < 0.9 | <2 |
| 11 | 0.70 | 1200 | 1.0 | 32.1 | 30.3 | < 0.9 | 1.8 |
| 12 | 0.70 | 1600 | 0.9 | 31.8 | 29.9 | < 0.9 | 1.9 |

As will appear from Table 29, for the test samples containing lactase, at the end of fermentation the level of lactose was very low and the level of glucose and galactose was very high as compared to the reference sample indicating high activity of the added lactase.
Fermented milk having a low lactose level and a high level of glucose and galactose has a much higher level of sweetness than fermented milk with a high lactose level and a low level of glucose and galactose like the reference samples. The reason for this is that glucose and galactose has a much higher sweetness than lactose.

**Sequence listing**

SEQ ID NO.: 1 shows the sequence of a mutant of SEQ ID NO. 4.
SEQ ID NO.: 2 shows the sequence of a mutant of SEQ ID NO. 4.
SEQ ID NO.: 3 shows the sequence of a lactase from *Bifidobacterium bifidum* DSM20215.
SEQ ID NO.: 4 shows the sequence of a lactase from *Bifidobacterium bifidum* NCI MB41171, the nucleotide sequence of which is listed in NCBI with the accession number DQ448279.

SEQ ID NO: 4 is discussed in the following references, wherein it is referred to as bbgIII:

Appl Microbiol Biotechnol (2007) 76:1365-1372, T K Goulas et al.
Appl Microbiol Biotechnol (2009) 82: 1079-1088, T Goulas et al.
Appl Microbiol Biotechnol (2009) 84:899-907, T Goulas et al.

**Claims**

1. A process for producing a fermented milk product comprising the steps of

   1) adding a starter culture comprising at least one lactic acid bacteria strain to a milk base,
   2) fermenting the milk for a period of time until a target pH is reached,
   3) wherein the starter culture comprises at least one lactose-deficient strain, which is capable of metabolizing a non-lactose carbohydrate, and
   4) adding a low pH stable lactase to the process either at the start, during or at the end of the fermentation step, wherein the low pH stable lactase retains its activity at a pH of 5.0 and a temperature of 37 °C at a level of at least 5 % as compared to its activity at the optimum pH of the lactase.

2. A process according to claim 1, wherein the low pH stable lactase retains its activity at a temperature of 10 °C and a pH of 6.0 at a level of at least 10 % as compared to its activity at the optimum temperature of the lactase.

3. A process according to claim 1 or 2, the wherein lactose-deficient strain is capable of metabolizing a non-lactose carbohydrate selected from the group consisting of sucrose, galactose and glucose.

4. A process according to any of the preceding claims, wherein non-lactose carbohydrate is added to the milk base at the start of the fermentation step.

5. A process according to claim 4, wherein the non-lactose carbohydrate is added to the milk base in an amount measured so as to become depleted and hence result in stopping the growth of lactic acid bacteria and in stopping the fermentation.

6. A process according to any of the preceding claims, wherein the low pH stable lactase is added to the milk base at the start of the fermentation step.

7. A process according to claim 6, wherein no non-lactose carbohydrate is added to the fermentation step, and wherein at least one lactose-deficient lactic acid strain of the starter culture is capable of metabolizing a carbohydrate selected from the group consisting of glucose and galactose.

8. A process according to any of claims 1-5, wherein the low pH stable lactase is added to the milk base at the end of the fermentation step.

9. A process according to any of claims 1-8, wherein the lactose-deficient strain is selected from the group consisting of lactose-deficient *Streptococcus thermophilus* and lactose-deficient *Lactobacillus delbrueckii* subsp. *bulgaricus.*

10. A process according to claim 9, wherein the lactose-deficient strain is selected from the group consisting of:

    (a) a *Streptococcus thermophilus* strain, which strain is:

    (i) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28952;
    (ii) or a strain derived from DSM 28952, wherein the derived strain is further characterized as having the ability to generate white colonies on a medium containing lactose and X-Gal;

    (b) a *Streptococcus thermophilus* strain, which strain is:

    (i) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28953;
(ii) or a strain derived from DSM 28953, wherein the derived strain is further characterized as having the ability to generate white colonies on a medium containing lactose and X-Gal;

(c) a *Lactobacillus delbrueckii* ssp. *bulgaricus* strain, which strain is:

(i) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28910;
(ii) or a strain derived from DSM 28910, wherein the derived strain is further characterized as having the ability to generate white colonies on a medium containing lactose and X-Gal.

11. A process according to claim 9 or 10, wherein the starter culture contains both at least one lactose-deficient *Streptococcus thermophilus* and at least one lactose-deficient *Lactobacillus delbrueckii* subsp. *bulgaricus.*

12. A process according to any of claims 9-11, wherein the starter culture contains both at least one *Streptococcus thermophilus* and at least one *Lactobacillus delbrueckii* subsp. *bulgaricus,* and wherein all *Streptococcus thermophilus* and all *Lactobacillus delbrueckii* subsp. *bulgaricus* strains are lactose-deficient.

13. Use in a process for producing a fermented milk product comprising the steps of

1) adding a starter culture comprising at least one lactic acid bacteria strain to a milk base,
2) fermenting the milk for a period of time until a target pH is reached,
of
3) the starter culture comprising at least one lactose-deficient strain, which is capable of metabolizing a non-lactose carbohydrate, and
4) a low pH stable lactase added to the process either at the start, during or at the end of the fermentation step, wherein the low pH stable lactase retains its activity at a pH of 5.0 and a temperature of 37 °C at a level of at least 5 % as compared to its activity at the optimum pH of the lactase.

14. Use according to claim 13 to increase the texture of the fermented milk product as compared to using lactose-deficient lactic acid bacteria and no lactase and as compared to using a low pH stable lactase and lactose-positive lactic acid bacteria.

**Patentansprüche**

1. Prozess zum Produzieren eines fermentierten Milchprodukts, umfassend die folgenden Schritte:

1) Hinzufügen einer Starterkultur, die zumindest einen Milchsäurebakterienstamm umfasst, zu einer Milchbasis,
2) Fermentierenlassen der Milch für einen Zeitraum, bis ein Ziel-pH-Wert erreicht ist,
3) wobei die Starterkultur zumindest einen laktosedefizienten Stamm umfasst, der in der Lage ist, ein Nicht-Laktose-Kohlenhydrat zu metabolisieren, und
4) Hinzufügen einer stabilen Laktase mit niedrigem pH-Wert zu dem Prozess, entweder zu Beginn, während oder gegen Ende des Fermentierungsschrittes, wobei die stabile Laktase mit niedrigem pH-Wert ihre Aktivität verglichen mit ihrer Aktivität bei dem optimalen pH-Wert der Laktase bei einem pH-Wert von 5,0 und einer Temperatur von 37 °C auf einem Niveau von zumindest 5 % hält.

2. Prozess nach Anspruch 1, wobei die stabile Laktase mit niedrigem pH-Wert ihre Aktivität verglichen mit ihrer Aktivität bei der optimalen Temperatur der Laktase bei einer Temperatur von 10 °C und einem pH-Wert von 6,0 auf einem Niveau von zumindest 10 % hält.

3. Prozess nach Anspruch 1 oder 2, wobei der laktosedefiziente Stamm in der Lage ist, ein Nicht-Laktose-Kohlenhydrat zu metabolisieren, das aus der Gruppe bestehend aus Saccharose, Galactose und Glucose ausgewählt ist.

4. Prozess nach einem der vorangehenden Ansprüche, wobei das Nicht-Laktose-Kohlenhydrat zu Beginn des Fermentierungsschrittes zu der Milchbasis hinzugefügt wird.

5. Prozess nach Anspruch 4, wobei das Nicht-Laktose-Kohlenhydrat in einer Menge, die abgemessen wird, um gesättigt zu werden und somit zu einem Anhalten des Wachstums der Milchsäurebakterien und einem Anhalten der Fermentierung zu führen, hinzugefügt wird.

6. Prozess nach einem der vorangehenden Ansprüche, wobei die stabile Laktase mit niedrigem pH-Wert zu Beginn des Fermentierungsschrittes zu der Milchbasis hinzugefügt wird.

7. Prozess nach Anspruch 6, wobei kein Nicht-Laktose-Kohlenhydrat zu dem Fermentierungsschritt hinzugefügt wird und wobei zumindest ein laktosedefizienter Milchsäurestamm der Starterkultur in der Lage ist, ein Kohlenhydrat zu metabolisieren, das aus der Gruppe bestehend aus Glucose und Galactose ausgewählt ist.

8. Prozess nach einem der Ansprüche 1-5, wobei die stabile Laktase mit niedrigem pH-Wert gegen Ende des Fermentierungsschrittes zu der Milchbasis hinzugefügt wird.

9. Prozess nach einem der Ansprüche 1-8, wobei der laktosedefiziente Stamm aus der Gruppe bestehend aus laktosedefizientem *Streptococcus thermophilus* und laktosedefizientem *Lactobacillus delbrueckii subsp. bulgaricus* ausgewählt ist.

10. Prozess nach Anspruch 9, wobei der laktosedefiziente Stamm aus der Gruppe ausgewählt ist, die aus Folgendem besteht:

   (a) einem *Streptococcus-thermophilus*-Stamm, wobei es sich bei dem Stamm um Folgendes handelt:

   (i) den Stamm, der am 12.06.2014 bei der DSMZ-Deutschen Sammlung von Mikroorganismen und Zellkulturen GmbH, Inhoffenstr. 7B, D-38124 Braunschweig, mit der Hinterlegungsnr. DSM 28952 hinterlegt wurde;
   (ii) oder einen Stamm, der von DSM 28952 abgeleitet wurde, wobei der abgeleitete Stamm ferner **dadurch gekennzeichnet ist, dass** er die Fähigkeit aufweist, weiße Kolonien auf einem Medium zu erzeugen, das Lactose und X-Gal enthält;

   (b) einem *Streptococcus-thermophilus*-Stamm, wobei es sich bei dem Stamm um Folgendes handelt:

   (i) den Stamm, der am 12.06.2014 bei der DSMZ-Deutschen Sammlung von Mikroorganismen und Zellkulturen GmbH, Inhoffenstr. 7B, D-38124 Braunschweig, mit der Hinterlegungsnr. DSM 28953 hinterlegt wurde;
   (ii) oder einen Stamm, der von DSM 28953 abgeleitet wurde, wobei der abgeleitete Stamm ferner **dadurch gekennzeichnet ist, dass** er die Fähigkeit aufweist, weiße Kolonien auf einem Medium zu erzeugen, das Lactose und X-Gal enthält;

   (c) einen *Lactobacillus-delbrueckii-ssp.-bulgaricus*-Stamm, wobei es sich bei dem Stamm um Folgendes handelt:

   (i) den Stamm, der am 12.06.2014 bei der DSMZ-Deutschen Sammlung von Mikroorganismen und Zellkulturen GmbH, Inhoffenstr. 7B, D-38124 Braunschweig, mit der Hinterlegungsnr. DSM 28910 hinterlegt wurde;
   (ii) oder einen Stamm, der von DSM 28910 abgeleitet wurde, wobei der abgeleitete Stamm ferner **dadurch gekennzeichnet ist, dass** er die Fähigkeit aufweist, weiße Kolonien auf einem Medium zu erzeugen, das Lactose und X-Gal enthält.

11. Prozess nach Anspruch 9 oder 10, wobei die Starterkultur sowohl zumindest einen laktosedefizienten *Streptococcus thermophilus* als auch zumindest einen laktosedefizienten *Lactobacillus delbrueckii subsp. bulgaricus* enthält.

12. Prozess nach einem der Ansprüche 9-11, wobei die Starterkultur sowohl zumindest einen *Streptococcus thermophilus* und zumindest einen *Lactobacillus delbrueckii subsp. bulgaricus* enthält und wobei alle *Streptococcus-thermophilus*- und alle *Lactobacillus-delbrueckii-subsp.-bulgaricus-Stämme* laktosedefizient sind.

13. Verwendung in einem Prozess zum Produzieren eines fermentierten Milchprodukts, umfassend die folgenden Schritte:

1) Hinzufügen einer Starterkultur, die zumindest einen Milchsäurebakterienstamm umfasst, zu einer Milchbasis,

2) Fermentierenlassen der Milch für einen Zeitraum, bis ein Ziel-pH-Wert

3) der Starterkultur erreicht ist, die zumindest einen laktosedefizienten Stamm umfasst, der in der Lage ist, ein Nicht-Laktose-Kohlenhydrat zu metabolisieren, und

4) wobei eine stabile Laktase mit niedrigem pH-Wert entweder zu Beginn, während oder gegen Ende des Fermentierungsschrittes zu dem Prozess hinzugefügt wird, wobei die stabile Laktase mit niedrigem pH-Wert ihre Aktivität verglichen mit ihrer Aktivität bei dem optimalen pH-Wert der Laktase bei einem pH-Wert von 5,0 und einer Temperatur von 37 °C auf einem Niveau von zumindest 5 % hält.

**14.** Verwendung nach Anspruch 13, um die Textur des fermentierten Milchprodukts verglichen mit Verwenden von laktosedefizienten Milchsäurebakterien und keiner Lactase und verglichen mit Verwendung einer stabilen Lactase mit niedrigem pH-Wert und laktosepositiven Milchsäurebakterien zu erhöhen.

## Revendications

**1.** Procédé de production d'un produit laitier fermenté comprenant les étapes

1) d'ajout d'une culture starter comprenant au moins une souche de bactéries lactiques à une base de lait,

2) de fermentation du lait pendant un certain temps jusqu'à ce qu'un pH cible soit atteint,

3) dans lequel la culture starter comprend au moins une souche déficiente en lactose, qui est capable de métaboliser un glucide sans lactose, et

4) d'ajout d'une lactase stable à pH bas au procédé, au début, pendant ou à la fin de l'étape de fermentation, dans lequel la lactase stable à pH bas maintient son activité à un pH de 5,0 et une température de 37 °C à un niveau d'au moins 5 % par rapport à son activité au pH optimal de la lactase.

**2.** Procédé selon la revendication 1, dans lequel la lactase stable à pH bas maintient son activité à une température de 10 °C et un pH de 6,0 à un niveau d'au moins 10 % par rapport à son activité à la température optimale de la lactase.

**3.** Procédé selon la revendication 1 ou 2, dans lequel la souche déficiente en lactose est capable de métaboliser un glucide sans lactose sélectionné parmi le groupe constitué de saccharose, de galactose et de glucose.

**4.** Procédé selon l'une quelconque des revendications précédentes, dans lequel un glucide sans lactose est ajouté à la base de lait au début de l'étape de fermentation.

**5.** Procédé selon la revendication 4, dans lequel le glucide sans lactose est ajouté à la base de lait en une quantité mesurée de manière à s'épuiser et donc entraîner l'arrêt de la croissance des bactéries lactiques et l'arrêt de la fermentation.

**6.** Procédé selon l'une quelconque des revendications précédentes, dans lequel la lactase stable à pH bas est ajoutée à la base de lait au début de l'étape de fermentation.

**7.** Procédé selon la revendication 6, dans lequel aucun glucide sans lactose n'est ajouté à l'étape de fermentation, et dans lequel au moins une souche d'acide lactique déficiente en lactose de la culture starter est capable de métaboliser un glucide sélectionné parmi le groupe constitué de glucose et de galactose.

**8.** Procédé selon l'une quelconque des revendications 1 à 5, dans lequel la lactase stable à pH bas est ajoutée à la base de lait à la fin de l'étape de fermentation.

**9.** Procédé selon l'une quelconque des revendications 1 à 8, dans lequel la souche déficiente en lactose est sélectionnée parmi le groupe constitué de *Streptococcus thermophilus* déficient en lactose et de *Lactobacillus delbrueckii* sous-espèce *bulgaricus* déficient en lactose.

**10.** Procédé selon la revendication 9, dans lequel la souche déficiente en lactose est sélectionnée parmi le groupe constitué :

(a) d'une souche de *Streptococcus thermophilus,* laquelle souche est :

(i) la souche déposée auprès de DSMZ-Deutsche Sammlung von Mikroorganismen und Zellkulturen GmbH, Inhoffenstr. 7B, D-38124 Braunschweig, le 12-06-2014 sous le n° d'entrée DSM 28952 ;
(ii) ou une souche dérivée de DSM 28952, dans lequel la souche dérivée est en outre **caractérisée** comme ayant la capacité à générer des colonies blanches sur un milieu contenant du lactose et X-Gal ;

(b) d'une souche de *Streptococcus thermophilus,* laquelle souche est :

(i) la souche déposée auprès de DSMZ-Deutsche Sammlung von Mikroorganismen und Zellkulturen GmbH, Inhoffenstr. 7B, D-38124 Braunschweig, le 12-06-2014 sous le n° d'entrée DSM 28953 ;
(ii) ou une souche dérivée de DSM 28953, dans lequel la souche dérivée est en outre **caractérisée** comme ayant la capacité à générer des colonies blanches sur un milieu contenant du lactose et X-Gal ;

(c) d'une souche de *Streptococcus delbrueckii* sous-espèce *bulgaricus,* laquelle souche est :

(i) la souche déposée auprès de DSMZ-Deutsche Sammlung von Mikroorganismen und Zellkulturen GmbH, Inhoffenstr. 7B, D-38124 Braunschweig, le 12-06-2014 sous le n° d'entrée DSM 28910 ;
(ii) ou une souche dérivée de DSM 28910, dans lequel la souche dérivée est en outre **caractérisée** comme ayant la capacité à générer des colonies blanches sur un milieu contenant du lactose et X-Gal.

11. Procédé selon la revendication 9 ou 10, dans lequel la culture starter contient à la fois au moins un *Streptococcus thermophilus* déficient en lactose et au moins un *Lactobacillus delbrueckii* sous-espèce *bulgaricus* déficient en lactose.

12. Procédé selon l'une quelconque des revendications 9 à 11, dans lequel la culture starter contient à la fois au moins un *Streptococcus thermophilus* et au moins un *Lactobacillus delbrueckii* sous-espèce *bulgaricus,* et dans lequel toutes les souches de *Streptococcus thermophilus* et toutes les souches de *Lactobacillus delbrueckii* sous-espèce *bulgaricus* sont déficientes en lactose.

13. Utilisation dans un procédé de production d'un produit laitier fermenté comprenant les étapes

1) d'ajout d'une culture starter comprenant au moins une souche de bactéries lactiques à une base de lait,
2) de fermentation du lait pendant un certain temps jusqu'à ce qu'un pH cible soit atteint,
3) la culture starter comprenant au moins une souche déficiente en lactose, qui est capable de métaboliser un glucide sans lactose, et
4) une lactase stable à pH bas ajoutée au procédé, au début, pendant ou à la fin de l'étape de fermentation, dans laquelle la lactase stable à pH bas maintient son activité à un pH de 5,0 et une température de 37 °C à un niveau d'au moins 5 % par rapport à son activité au pH optimal de la lactase.

14. Utilisation selon la revendication 13 pour augmenter la texture du produit laitier fermenté par rapport à l'utilisation de bactéries lactiques déficientes en lactose et sans lactase et par rapport à l'utilisation d'une lactase stable à pH bas et de bactéries lactiques positives pour le lactose.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009071539 A
- WO 2013160413 A
- EP 2957180 A1

**Non-patent literature cited in the description**

- **NEEDLEMAN ; WUNSCH.** *J. Mol. Biol.,* 1970, vol. 48, 443-453
- **RICE et al.** EMBOSS: The European Molecular Biology Open Software Suite. *Trends in Genetics,* 2000, vol. 16, 276-277
- **WERNER, W. et al.** *Z. analyt. Chem.,* 1970, vol. 252, 224
- **T K GOULAS.** *Appl Microbiol Biotechnol,* 2007, vol. 76, 1365-1372
- **T GOULAS.** *Appl Microbiol Biotechnol,* 2009, vol. 82, 1079-1088
- **T GOULAS.** *Appl Microbiol Biotechnol,* 2009, vol. 84, 899-907

SEQUENCE LISTING

<110> Chr. Hansen A/S

<120> Process for producing a fermented milk product

<130> P6170PC00

<160> 4

<170> PatentIn version 3.5

<210> 1
<211> 1931
<212> PRT
<213> Bifidobacterium bifidum

<400> 1

```
Met Lys Lys Pro Leu Gly Lys Ile Val Ala Ser Thr Ala Leu Leu Ile
1               5                   10                  15

Ser Val Ala Phe Ser Ser Ser Ile Ala Ser Ala Ala Val Glu Asp Ala
                20                  25                  30

Thr Arg Ser Asp Ser Thr Thr Gln Met Ser Ser Thr Pro Glu Val Ala
            35                  40                  45

Tyr Ser Ser Ala Val Asp Ser Lys Gln Asn Arg Thr Ser Asp Phe Asp
        50                  55                  60

Ala Asn Trp Lys Phe Met Leu Ser Asp Ser Val Gln Ala Gln Asp Pro
65                  70                  75                  80

Ala Phe Asp Asp Ser Ala Trp Gln Gln Val Asp Leu Pro His Asp Tyr
                85                  90                  95

Ser Ile Thr Gln Lys Tyr Ser Gln Ser Asn Glu Ala Glu Ser Ala Tyr
            100                 105                 110

Leu Pro Gly Gly Thr Gly Trp Tyr Arg Lys Ser Phe Thr Ile Asp Arg
        115                 120                 125

Asp Leu Ala Gly Lys Arg Ile Ala Ile Asn Phe Asp Gly Val Tyr Met
        130                 135                 140

Asn Ala Thr Val Trp Phe Asn Gly Val Lys Leu Gly Thr His Pro Tyr
145                 150                 155                 160

Gly Tyr Ser Pro Phe Ser Phe Asp Leu Thr Gly Asn Ala Lys Phe Gly
                165                 170                 175
```

Gly Glu Asn Thr Ile Val Val Lys Val Glu Asn Arg Leu Pro Ser Ser
            180                 185                 190

Arg Trp Tyr Ser Gly Ser Gly Ile Tyr Arg Asp Val Thr Leu Thr Val
            195                 200                 205

Thr Asp Gly Val His Val Gly Asn Asn Gly Val Ala Ile Lys Thr Pro
            210                 215                 220

Ser Leu Ala Thr Gln Asn Gly Gly Asp Val Thr Met Asn Leu Thr Thr
225                 230                 235                 240

Lys Val Ala Asn Asp Thr Glu Ala Ala Ala Asn Ile Thr Leu Lys Gln
                245                 250                 255

Thr Val Phe Pro Lys Gly Gly Lys Thr Asp Ala Ala Ile Gly Thr Val
            260                 265                 270

Thr Thr Ala Ser Lys Ser Ile Ala Ala Gly Ala Ser Ala Asp Val Thr
            275                 280                 285

Ser Thr Ile Thr Ala Ala Ser Pro Lys Leu Trp Ser Ile Lys Asn Pro
            290                 295                 300

Asn Leu Tyr Thr Val Arg Thr Glu Val Leu Asn Gly Gly Lys Val Leu
305                 310                 315                 320

Asp Thr Tyr Asp Thr Glu Tyr Gly Phe Arg Trp Thr Gly Phe Asp Ala
            325                 330                 335

Thr Ser Gly Phe Ser Leu Asn Gly Glu Lys Val Lys Leu Lys Gly Val
            340                 345                 350

Ser Met His His Asp Gln Gly Ser Leu Gly Ala Val Ala Asn Arg Arg
            355                 360                 365

Ala Ile Glu Arg Gln Val Glu Ile Leu Gln Lys Met Gly Val Asn Ser
            370                 375                 380

Ile Arg Thr Thr His Asn Pro Ala Ala Lys Ala Leu Ile Asp Val Cys
385                 390                 395                 400

Asn Glu Lys Gly Val Leu Val Val Glu Glu Val Phe Asp Met Trp Asn
            405                 410                 415

Arg Ser Lys Asn Gly Asn Thr Glu Asp Tyr Gly Lys Trp Phe Gly Gln
            420                 425                 430

```
Ala Ile Ala Gly Asp Asn Ala Val Leu Gly Gly Asp Lys Asp Glu Thr
    435             440             445

Trp Ala Lys Phe Asp Leu Thr Ser Thr Ile Asn Arg Asp Arg Asn Ala
    450             455             460

Pro Ser Val Ile Met Trp Ser Leu Gly Asn Glu Met Met Glu Gly Ile
465             470             475             480

Ser Gly Ser Val Ser Gly Phe Pro Ala Thr Ser Ala Lys Leu Val Ala
                485             490             495

Trp Thr Lys Ala Ala Asp Ser Thr Arg Pro Met Thr Tyr Gly Asp Asn
        500             505             510

Lys Ile Lys Ala Asn Trp Asn Glu Ser Asn Thr Met Gly Asp Asn Leu
        515             520             525

Thr Ala Asn Gly Gly Val Val Gly Thr Asn Tyr Ser Asp Gly Ala Asn
    530             535             540

Tyr Asp Lys Ile Arg Thr Thr His Pro Ser Trp Ala Ile Tyr Gly Ser
545             550             555             560

Glu Thr Ala Ser Ala Ile Asn Ser Arg Gly Ile Tyr Asn Arg Thr Thr
                565             570             575

Gly Gly Ala Gln Ser Ser Asp Lys Gln Leu Thr Ser Tyr Asp Asn Ser
            580             585             590

Ala Val Gly Trp Gly Ala Val Ala Ser Ser Ala Trp Tyr Asp Val Val
            595             600             605

Gln Arg Asp Phe Val Ala Gly Thr Tyr Val Trp Thr Gly Phe Asp Tyr
    610             615             620

Leu Gly Glu Pro Thr Pro Trp Asn Gly Thr Gly Ser Gly Ala Val Gly
625             630             635             640

Ser Trp Pro Ser Pro Lys Asn Ser Tyr Phe Gly Ile Val Asp Thr Ala
            645             650             655

Gly Phe Pro Lys Asp Thr Tyr Tyr Phe Tyr Gln Ser Gln Trp Asn Asp
            660             665             670

Asp Val His Thr Leu His Ile Leu Pro Ala Trp Asn Glu Asn Val Val
        675             680             685
```

32

```
Ala Lys Gly Ser Gly Asn Asn Val Pro Val Val Val Tyr Thr Asp Ala
    690             695             700

Ala Lys Val Lys Leu Tyr Phe Thr Pro Lys Gly Ser Thr Glu Lys Arg
705             710             715                 720

Leu Ile Gly Glu Lys Ser Phe Thr Lys Lys Thr Thr Ala Ala Gly Tyr
                725             730                 735

Thr Tyr Gln Val Tyr Glu Gly Ser Asp Lys Asp Ser Thr Ala His Lys
            740             745             750

Asn Met Tyr Leu Thr Trp Asn Val Pro Trp Ala Glu Gly Thr Ile Ser
        755             760             765

Ala Glu Ala Tyr Asp Glu Asn Asn Arg Leu Ile Pro Glu Gly Ser Thr
770             775             780

Glu Gly Asn Ala Ser Val Thr Thr Thr Gly Lys Ala Ala Lys Leu Lys
785             790             795                 800

Ala Asp Ala Asp Arg Lys Thr Ile Thr Ala Asp Gly Lys Asp Leu Ser
            805             810             815

Tyr Ile Glu Val Asp Val Thr Asp Ala Asn Gly His Ile Val Pro Asp
        820             825             830

Ala Ala Asn Arg Val Thr Phe Asp Val Lys Gly Ala Gly Lys Leu Val
        835             840             845

Gly Val Asp Asn Gly Ser Ser Pro Asp His Asp Ser Tyr Gln Ala Asp
    850             855             860

Asn Arg Lys Ala Phe Ser Gly Lys Val Leu Ala Ile Val Gln Ser Thr
865             870             875                 880

Lys Glu Ala Gly Glu Ile Thr Val Thr Ala Lys Ala Asp Gly Leu Gln
                885             890                 895

Ser Ser Thr Val Lys Ile Ala Thr Thr Ala Val Pro Gly Thr Ser Thr
            900             905             910

Glu Lys Thr Val Arg Ser Phe Tyr Tyr Ser Arg Asn Tyr Tyr Val Lys
        915             920             925

Thr Gly Asn Lys Pro Ile Leu Pro Ser Asp Val Glu Val Arg Tyr Ser
```

33

```
                930                      935                        940


       Asp Gly Thr Ser Asp Arg Gln Asn Val Thr Trp Asp Ala Val Ser Asp
       945                 950                   955                    960


       Asp Gln Ile Ala Lys Ala Gly Ser Phe Ser Val Ala Gly Thr Val Ala
                       965                   970                    975


       Gly Gln Lys Ile Ser Val Arg Val Thr Met Ile Asp Glu Ile Gly Ala
                       980                   985                    990


       Leu Leu Asn Tyr Ser Ala Ser Thr  Pro Val Gly Thr Pro  Ala Val Leu
                  995                   1000                  1005


       Pro Gly  Ser Arg Pro Ala Val  Leu Pro Asp Gly Thr  Val Thr Ser
           1010                  1015                  1020


       Ala Asn  Phe Ala Val His Trp  Thr Lys Pro Ala Asp  Thr Val Tyr
           1025                  1030                  1035


       Asn Thr  Ala Gly Thr Val Lys  Val Pro Gly Thr Ala  Thr Val Phe
           1040                  1045                  1050


       Gly Lys  Glu Phe Lys Val Thr  Ala Thr Ile Arg Val  Gln Arg Ser
           1055                  1060                  1065


       Gln Val  Thr Ile Gly Ser Ser  Val Ser Gly Asn Ala  Leu Arg Leu
           1070                  1075                  1080


       Thr Gln  Asn Ile Pro Ala Asp  Lys Gln Ser Asp Thr  Leu Asp Ala
           1085                  1090                  1095


       Ile Lys  Asp Gly Ser Thr Thr  Val Asp Ala Asn Thr  Gly Gly Gly
           1100                  1105                  1110


       Ala Asn  Pro Ser Ala Trp Thr  Asn Trp Ala Tyr Ser  Lys Ala Gly
           1115                  1120                  1125


       His Asn  Thr Ala Glu Ile Thr  Phe Glu Tyr Ala Thr  Glu Gln Gln
           1130                  1135                  1140


       Leu Gly  Gln Ile Val Met Tyr  Phe Phe Arg Asp Ser  Asn Ala Val
           1145                  1150                  1155


       Arg Phe  Pro Asp Ala Gly Lys  Thr Lys Ile Gln Ile  Ser Ala Asp
           1160                  1165                  1170
```

```
Gly Lys Asn Trp Thr Asp Leu Ala Ala Thr Glu Thr Ile Ala Ala
    1175            1180            1185

Gln Glu Ser Ser Asp Arg Val Lys Pro Tyr Thr Tyr Asp Phe Ala
    1190            1195            1200

Pro Val Gly Ala Thr Phe Val Lys Val Thr Val Thr Asn Ala Asp
    1205            1210            1215

Thr Thr Thr Pro Ser Gly Val Val Cys Ala Gly Leu Thr Glu Ile
    1220            1225            1230

Glu Leu Lys Thr Ala Thr Ser Lys Phe Val Thr Asn Thr Ser Ala
    1235            1240            1245

Ala Leu Ser Ser Leu Thr Val Asn Gly Thr Lys Val Ser Asp Ser
    1250            1255            1260

Val Leu Ala Ala Gly Ser Tyr Asn Thr Pro Ala Ile Ile Ala Asp
    1265            1270            1275

Val Lys Ala Glu Gly Glu Gly Asn Ala Ser Val Thr Val Leu Pro
    1280            1285            1290

Ala His Asp Asn Val Ile Arg Val Ile Thr Glu Ser Glu Asp His
    1295            1300            1305

Val Thr Arg Lys Thr Phe Thr Ile Asn Leu Gly Thr Glu Gln Glu
    1310            1315            1320

Phe Pro Ala Asp Ser Asp Glu Arg Asp Tyr Pro Ala Ala Asp Met
    1325            1330            1335

Thr Val Thr Val Gly Ser Glu Gln Thr Ser Gly Thr Ala Thr Glu
    1340            1345            1350

Gly Pro Lys Lys Phe Ala Val Asp Gly Asn Thr Ser Thr Tyr Trp
    1355            1360            1365

His Ser Asn Trp Thr Pro Thr Thr Val Asn Asp Leu Trp Ile Ala
    1370            1375            1380

Phe Glu Leu Gln Lys Pro Thr Lys Leu Asp Ala Leu Arg Tyr Leu
    1385            1390            1395

Pro Arg Pro Ala Gly Ser Lys Asn Gly Ser Val Thr Glu Tyr Lys
    1400            1405            1410
```

35

```
Val Gln Val Ser Asp Asp Gly   Thr Asn Trp Thr Asp   Ala Gly Ser
    1415            1420               1425

Gly Thr Trp Thr Thr Asp Tyr   Gly Trp Lys Leu Ala   Glu Phe Asn
    1430            1435               1440

Gln Pro Val Thr Thr Lys His   Val Arg Leu Lys Ala   Val His Thr
    1445            1450               1455

Tyr Ala Asp Ser Gly Asn Asp   Lys Phe Met Ser Ala   Ser Glu Ile
    1460            1465               1470

Arg Leu Arg Lys Ala Val Asp   Thr Thr Asp Ile Ser   Gly Ala Thr
    1475            1480               1485

Val Thr Val Pro Ala Lys Leu   Thr Val Asp Arg Val   Asp Ala Asp
    1490            1495               1500

His Pro Ala Thr Phe Ala Thr   Lys Asp Val Thr Val   Thr Leu Gly
    1505            1510               1515

Asp Ala Thr Leu Arg Tyr Gly   Val Asp Tyr Leu Leu   Asp Tyr Ala
    1520            1525               1530

Gly Asn Thr Ala Val Gly Lys   Ala Thr Val Thr Val   Arg Gly Ile
    1535            1540               1545

Asp Lys Tyr Ser Gly Thr Val   Ala Lys Thr Phe Thr   Ile Glu Leu
    1550            1555               1560

Lys Asn Ala Pro Ala Pro Glu   Pro Thr Leu Thr Ser   Val Ser Val
    1565            1570               1575

Lys Thr Lys Pro Ser Lys Leu   Thr Tyr Val Val Gly   Asp Ala Phe
    1580            1585               1590

Asp Pro Ala Gly Leu Val Leu   Gln Leu Asn Tyr Asp   Asp Asp Ser
    1595            1600               1605

Thr Gly Thr Val Thr Trp Asn   Thr Gln Thr Ala Gly   Asp Phe Thr
    1610            1615               1620

Phe Lys Pro Ala Leu Asp Ala   Lys Leu Lys Val Thr   Asp Lys Thr
    1625            1630               1635

Val Thr Val Thr Tyr Gln Gly   Lys Ser Ala Val Ile   Asp Ile Thr
    1640            1645               1650
```

```
Val Ser Gln Pro Ala Pro Thr  Val Ser Lys Thr Asp  Leu Asp Lys
    1655            1660             1665


Ala Ile Lys Ala Ile Glu Ala  Lys Asn Pro Asp Ser  Ser Lys Tyr
    1670            1675             1680


Thr Ala Asp Ser Trp Lys Thr  Phe Ala Asp Ala Met  Ala His Ala
    1685            1690             1695


Lys Ala Val Ile Ala Asp Asp  Ser Ala Thr Gln Gln  Asp Val Asp
    1700            1705             1710


Asn Ala Leu Lys Ala Leu Thr  Asp Ala Tyr Ala Gly  Leu Thr Glu
    1715            1720             1725


Lys Thr Pro Glu Pro Ala Pro  Val Ser Lys Ser Glu  Leu Asp Lys
    1730            1735             1740


Lys Ile Lys Ala Ile Glu Ala  Glu Lys Leu Asp Gly  Ser Lys Tyr
    1745            1750             1755


Thr Ala Glu Ser Trp Lys Ala  Phe Glu Thr Ala Leu  Ala His Ala
    1760            1765             1770


Lys Ala Val Ile Ala Ser Asp  Ser Ala Thr Gln Gln  Asn Val Asp
    1775            1780             1785


Ala Ala Leu Gly Ala Leu Thr  Ser Ala Arg Asp Gly  Leu Thr Glu
    1790            1795             1800


Lys Gly Glu Val Lys Pro Asp  Pro Lys Pro Glu Pro  Gly Thr Val
    1805            1810             1815


Asp Lys Ala Ala Leu Asp Lys  Ala Val Lys Lys Val  Glu Ala Glu
    1820            1825             1830


Lys Leu Asp Gly Ser Lys Tyr  Thr Ala Asp Ser Trp  Lys Ala Phe
    1835            1840             1845


Glu Thr Ala Leu Ala His Ala  Lys Ala Val Ile Gly  Asn Ala Asn
    1850            1855             1860


Ser Thr Gln Phe Asp Ile Asp  Asn Ala Leu Ser Met  Leu Asn Asp
    1865            1870             1875


Ala Arg Ala Ala Leu Lys Glu  Lys Pro Gly Arg Ile  Ile Ala Ile
```

37

```
                1880                    1885                    1890


        Ile Asp  Gly Ser Ala Leu Ser  Lys Thr Gly Ala Ser  Val Ala Ile
            1895                    1900                    1905


        Ile Ala  Ser Val Ala Ala Ala  Met Leu Ala Val Gly  Ala Gly Val
            1910                    1915                    1920


        Met Ala  Leu Arg Arg Lys Arg  Ser
            1925                    1930
```

<210> 2
<211> 1341
<212> PRT
<213> Bifidobacterium bifidum

<400> 2

```
Met Lys Lys Pro Leu Gly Lys Ile Val Ala Ser Thr Ala Leu Leu Ile
1               5                   10                  15

Ser Val Ala Phe Ser Ser Ser Ile Ala Ser Ala Ile Glu Asp Ala Thr
            20                  25                  30

Arg Ser Asp Ser Thr Thr Gln Met Ser Ser Thr Pro Glu Val Ala Tyr
        35                  40                  45

Ser Ser Ala Val Asp Ser Lys Gln Asn Arg Thr Ser Asp Phe Asp Ala
        50                  55                  60

Asn Trp Lys Phe Met Leu Ser Asp Ser Val Gln Ala Gln Asp Pro Ala
65                  70                  75                  80

Phe Asp Asp Ser Ala Trp Gln Gln Val Asp Leu Pro His Asp Tyr Ser
            85                  90                  95

Ile Thr Gln Lys Tyr Ser Gln Ser Asn Glu Ala Glu Ser Ala Tyr Leu
            100                 105                 110

Pro Gly Gly Thr Gly Trp Tyr Arg Lys Ser Phe Thr Ile Asp Arg Asp
        115                 120                 125

Leu Ala Gly Lys Arg Ile Ala Ile Asn Phe Asp Gly Val Tyr Met Asn
    130                 135                 140

Ala Thr Val Trp Phe Asn Gly Val Lys Leu Gly Thr His Pro Tyr Gly
145                 150                 155                 160

Tyr Ser Pro Phe Ser Phe Asp Leu Thr Gly Asn Ala Lys Phe Gly Gly
```

                    165                    170                    175

Glu Asn Thr Ile Val Val Lys Val Glu Asn Arg Leu Pro Ser Ser Arg
            180                    185                    190

Trp Tyr Ser Gly Ser Gly Ile Tyr Arg Asp Val Thr Leu Thr Val Thr
            195                    200                    205

Asp Gly Val His Val Gly Asn Asn Gly Val Ala Ile Lys Thr Pro Ser
    210                    215                    220

Leu Ala Thr Gln Asn Gly Gly Asp Val Thr Met Asn Leu Thr Thr Lys
225                    230                    235                    240

Val Ala Asn Asp Thr Glu Ala Ala Ala Asn Ile Thr Leu Lys Gln Thr
                245                    250                    255

Val Phe Pro Lys Gly Gly Lys Thr Asp Ala Ala Ile Gly Thr Val Thr
            260                    265                    270

Thr Ala Ser Lys Ser Ile Ala Ala Gly Ala Ser Ala Asp Val Thr Ser
            275                    280                    285

Thr Ile Thr Ala Ala Ser Pro Lys Leu Trp Ser Ile Lys Asn Pro Asn
    290                    295                    300

Leu Tyr Thr Val Arg Thr Glu Val Leu Asn Gly Gly Lys Val Leu Asp
305                    310                    315                    320

Thr Tyr Asp Thr Glu Tyr Gly Phe Arg Trp Thr Gly Phe Asp Ala Thr
                325                    330                    335

Ser Gly Phe Ser Leu Asn Gly Glu Lys Val Lys Leu Lys Gly Val Ser
            340                    345                    350

Met His His Asp Gln Gly Ser Leu Gly Ala Val Ala Asn Arg Arg Ala
            355                    360                    365

Ile Glu Arg Gln Val Glu Ile Leu Gln Lys Met Gly Val Asn Ser Ile
    370                    375                    380

Arg Thr Thr His Asn Pro Ala Ala Lys Ala Leu Ile Asp Val Cys Asn
385                    390                    395                    400

Glu Lys Gly Val Leu Val Val Glu Glu Val Phe Asp Met Trp Asn Arg
            405                    410                    415

```
Ser Lys Asn Gly Asn Thr Glu Asp Tyr Gly Lys Trp Phe Gly Gln Ala
        420                 425                 430

Ile Ala Gly Asp Asn Ala Val Leu Gly Gly Asp Lys Asp Glu Thr Trp
        435                 440                 445

Ala Lys Phe Asp Leu Thr Ser Thr Ile Asn Arg Asp Arg Asn Ala Pro
        450                 455                 460

Ser Val Ile Met Trp Ser Leu Gly Asn Glu Met Met Glu Gly Ile Ser
465                 470                 475                 480

Gly Ser Val Ser Gly Phe Ser Ala Thr Ser Ala Lys Leu Val Ala Trp
                485                 490                 495

Thr Lys Ala Ala Asp Ser Thr Arg Pro Met Thr Tyr Gly Asp Asn Lys
        500                 505                 510

Ile Lys Ala Asn Trp Asn Glu Ser Asn Thr Met Gly Asp Asn Leu Thr
        515                 520                 525

Ala Asn Gly Gly Val Val Gly Thr Asn Tyr Ser Asp Gly Ala Asn Tyr
        530                 535                 540

Asp Lys Ile Arg Thr Thr His Pro Ser Trp Ala Ile Tyr Gly Ser Glu
545                 550                 555                 560

Thr Ala Ser Ala Ile Asn Ser Arg Gly Ile Tyr Asn Arg Thr Thr Gly
                565                 570                 575

Gly Ala Gln Ser Ser Asp Lys Gln Leu Thr Ser Tyr Asp Asn Ser Ala
                580                 585                 590

Val Gly Trp Gly Ala Val Ala Ser Ser Ala Trp Tyr Asp Val Val Gln
        595                 600                 605

Arg Asp Phe Val Ala Gly Thr Tyr Val Trp Thr Gly Phe Asp Tyr Leu
        610                 615                 620

Gly Glu Pro Thr Pro Trp Asn Gly Thr Gly Ser Gly Ala Val Gly Ser
625                 630                 635                 640

Trp Pro Ser Pro Lys Asn Ser Tyr Phe Gly Ile Val Asp Thr Ala Gly
                645                 650                 655

Phe Pro Lys Asp Thr Tyr Tyr Phe Tyr Gln Ser Gln Trp Asn Asp Asp
        660                 665                 670
```

41

Val His Thr Leu His Ile Leu Pro Ala Trp Asn Glu Asn Val Val Ala
675 680 685

Lys Gly Ser Gly Asn Asn Val Pro Val Val Val Tyr Thr Asp Ala Ala
690 695 700

Lys Val Lys Leu Tyr Phe Thr Pro Lys Gly Ser Thr Glu Gln Arg Leu
705 710 715 720

Ile Gly Glu Lys Ser Phe Thr Lys Lys Thr Thr Ala Ala Gly Tyr Thr
725 730 735

Tyr Gln Val Tyr Glu Gly Ser Asp Lys Asp Ser Thr Ala His Lys Asn
740 745 750

Met Tyr Leu Thr Trp Asn Val Pro Trp Ala Glu Gly Thr Ile Ser Ala
755 760 765

Glu Ala Tyr Asp Glu Asn Asn Arg Leu Ile Pro Glu Gly Ser Thr Glu
770 775 780

Gly Asn Ala Ser Val Thr Thr Thr Gly Lys Ala Ala Lys Leu Lys Ala
785 790 795 800

Asp Ala Asp Arg Lys Thr Ile Thr Ala Asp Gly Lys Asp Leu Ser Tyr
805 810 815

Ile Glu Val Asp Val Thr Asp Ala Asn Gly His Ile Val Pro Asp Ala
820 825 830

Ala Asn Arg Val Thr Phe Asp Val Lys Gly Ala Gly Lys Leu Val Gly
835 840 845

Val Asp Asn Gly Ser Ser Pro Asp His Asp Ser Tyr Gln Ala Asp Asn
850 855 860

Arg Lys Ala Phe Ser Gly Lys Val Leu Ala Ile Val Gln Ser Thr Lys
865 870 875 880

Glu Ala Gly Glu Ile Thr Val Thr Ala Lys Ala Asp Gly Leu Gln Ser
885 890 895

Ser Thr Val Lys Ile Ala Thr Thr Ala Val Pro Gly Thr Ser Thr Glu
900 905 910

Lys Thr Val Arg Ser Phe Tyr Tyr Ser Arg Asn Tyr Tyr Val Lys Thr
915 920 925

42

```
Gly Asn Lys Pro Ile Leu Pro Ser Asp Val Glu Val Arg Tyr Ser Asp
    930                 935                 940

Gly Thr Ser Asp Arg Gln Asn Val Thr Trp Asp Ala Val Ser Asp Asp
945                 950                 955                 960

Gln Ile Ala Lys Ala Gly Ser Phe Ser Val Ala Gly Thr Val Ala Gly
                965                 970                 975

Gln Lys Ile Ser Val Arg Val Thr Met Ile Asp Glu Ile Gly Ala Leu
                980                 985                 990

Leu Asn Tyr Ser Ala Ser Thr Pro  Val Gly Thr Pro Ala  Val Leu Pro
            995                 1000                1005

Gly Ser  Arg Pro Ala Val Leu  Pro Asp Gly Thr Val  Thr Ser Ala
    1010                1015                1020

Asn Phe  Ala Val His Trp Thr  Lys Pro Ala Asp Thr  Val Tyr Asn
    1025                1030                1035

Thr Ala  Gly Thr Val Lys Val  Pro Gly Thr Ala Thr  Val Phe Gly
    1040                1045                1050

Lys Glu  Phe Lys Val Thr Ala  Thr Ile Arg Val Gln  Arg Ser Gln
    1055                1060                1065

Val Thr  Ile Gly Ser Ser Val  Ser Gly Asn Ala Leu  Arg Leu Thr
    1070                1075                1080

Gln Asn  Ile Pro Ala Asp Lys  Gln Ser Asp Thr Leu  Asp Ala Ile
    1085                1090                1095

Lys Asp  Gly Ser Thr Thr Val  Asp Ala Asn Thr Gly  Gly Gly Ala
    1100                1105                1110

Asn Pro  Ser Ala Trp Thr Asn  Trp Ala Tyr Ser Lys  Ala Gly His
    1115                1120                1125

Asn Thr  Ala Glu Ile Thr Phe  Glu Tyr Ala Thr Glu  Gln Gln Leu
    1130                1135                1140

Gly Gln  Ile Val Met Tyr Phe  Phe Arg Asp Ser Asn  Ala Val Arg
    1145                1150                1155

Phe Pro  Asp Ala Gly Lys Thr  Lys Ile Gln Ile Ser  Ala Asp Gly
```

```
        1160                    1165                    1170

Lys Asn Trp Thr Asp Leu Ala Ala Thr Glu Thr Ile Ala Ala Gln
    1175            1180            1185

Glu Ser Ser Asp Arg Val Lys Pro Tyr Thr Tyr Asp Phe Ala Pro
    1190            1195            1200

Val Gly Ala Thr Phe Val Arg Val Thr Val Thr Asn Ala Asp Thr
    1205            1210            1215

Thr Thr Pro Ser Gly Val Val Cys Ala Gly Leu Thr Glu Ile Glu
    1220            1225            1230

Leu Lys Thr Ala Thr Ser Lys Phe Val Ala Asn Thr Ser Ala Ala
    1235            1240            1245

Leu Ser Ser Leu Thr Val Asn Gly Thr Lys Val Ser Asp Ser Val
    1250            1255            1260

Leu Ala Ala Gly Ser Tyr Asn Thr Pro Ala Ile Ile Ala Asp Val
    1265            1270            1275

Lys Ala Glu Gly Glu Gly Asn Ala Ser Val Thr Val Leu Pro Ala
    1280            1285            1290

His Asp Asn Val Ile Arg Val Ile Thr Glu Ser Glu Asp His Val
    1295            1300            1305

Thr Arg Lys Thr Phe Thr Ile Asn Leu Gly Thr Glu Gln Glu Phe
    1310            1315            1320

Pro Ala Asp Ser Asp Glu Arg Asp Gln His Gln His Gln His Gln
    1325            1330            1335

His Gln Gln
    1340
```

<210> 3
<211> 1752
<212> PRT
<213> Bifidobacterium bifidum

<400> 3

44

Met Ala Val Arg Arg Leu Gly Gly Arg Ile Val Ala Phe Ala Ala Thr
1           5                 10              15

Val Ala Leu Ser Ile Pro Leu Gly Leu Leu Thr Asn Ser Ala Trp Ala

```
                      20                          25                          30

          Val Glu Asp Ala Thr Arg Ser Asp Ser Thr Thr Gln Met Ser Ser Thr
                  35                  40                  45

          Pro Glu Val Val Tyr Ser Ser Ala Val Asp Ser Lys Gln Asn Arg Thr
                  50                  55                  60

          Ser Asp Phe Asp Ala Asn Trp Lys Phe Met Leu Ser Asp Ser Val Gln
          65                  70                  75                  80

          Ala Gln Asp Pro Ala Phe Asp Asp Ser Ala Trp Gln Gln Val Asp Leu
                          85                  90                  95

          Pro His Asp Tyr Ser Ile Thr Gln Lys Tyr Ser Gln Ser Asn Glu Ala
                      100                 105                 110

          Glu Ser Ala Tyr Leu Pro Gly Gly Thr Gly Trp Tyr Arg Lys Ser Phe
                      115                 120                 125

          Thr Ile Asp Arg Asp Leu Ala Gly Lys Arg Ile Ala Ile Asn Phe Asp
                  130                 135                 140

          Gly Val Tyr Met Asn Ala Thr Val Trp Phe Asn Gly Val Lys Leu Gly
          145                 150                 155                 160

          Thr His Pro Tyr Gly Tyr Ser Pro Phe Ser Phe Asp Leu Thr Gly Asn
                          165                 170                 175

          Ala Lys Phe Gly Gly Glu Asn Thr Ile Val Val Lys Val Glu Asn Arg
                      180                 185                 190

          Leu Pro Ser Ser Arg Trp Tyr Ser Gly Ser Gly Ile Tyr Arg Asp Val
                      195                 200                 205

          Thr Leu Thr Val Thr Asp Gly Val His Val Gly Asn Asn Gly Val Ala
                  210                 215                 220

          Ile Lys Thr Pro Ser Leu Ala Thr Gln Asn Gly Gly Asp Val Thr Met
          225                 230                 235                 240

          Asn Leu Thr Thr Lys Val Ala Asn Asp Thr Glu Ala Ala Ala Asn Ile
                          245                 250                 255

          Thr Leu Lys Gln Thr Val Phe Pro Lys Gly Gly Lys Thr Asp Ala Ala
                      260                 265                 270
```

46

```
Ile Gly Thr Val Thr Thr Ala Ser Lys Ser Ile Ala Ala Gly Ala Ser
        275             280             285

Ala Asp Val Thr Ser Thr Ile Thr Ala Ala Ser Pro Lys Leu Trp Ser
        290             295             300

Ile Lys Asn Pro Asn Leu Tyr Thr Val Arg Thr Glu Val Leu Asn Gly
305             310             315             320

Gly Lys Val Leu Asp Thr Tyr Asp Thr Glu Tyr Gly Phe Arg Trp Thr
        325             330             335

Gly Phe Asp Ala Thr Ser Gly Phe Ser Leu Asn Gly Glu Lys Val Lys
        340             345             350

Leu Lys Gly Val Ser Met His His Asp Gln Gly Ser Leu Gly Ala Val
        355             360             365

Ala Asn Arg Arg Ala Ile Glu Arg Gln Val Glu Ile Leu Gln Lys Met
        370             375             380

Gly Val Asn Ser Ile Arg Thr Thr His Asn Pro Ala Ala Lys Ala Leu
385             390             395             400

Ile Asp Val Cys Asn Glu Lys Gly Val Leu Val Val Glu Glu Val Phe
            405             410             415

Asp Met Trp Asn Arg Ser Lys Asn Gly Asn Thr Glu Asp Tyr Gly Lys
            420             425             430

Trp Phe Gly Gln Ala Ile Ala Gly Asp Asn Ala Val Leu Gly Gly Asp
        435             440             445

Lys Asp Glu Thr Trp Ala Lys Phe Asp Leu Thr Ser Thr Ile Asn Arg
        450             455             460

Asp Arg Asn Ala Pro Ser Val Ile Met Trp Ser Leu Gly Asn Glu Met
465             470             475             480

Met Glu Gly Ile Ser Gly Ser Val Ser Gly Phe Pro Ala Thr Ser Ala
            485             490             495

Lys Leu Val Ala Trp Thr Lys Ala Ala Asp Ser Thr Arg Pro Met Thr
        500             505             510

Tyr Gly Asp Asn Lys Ile Lys Ala Asn Trp Asn Glu Ser Asn Thr Met
        515             520             525
```

47

```
Gly Asp Asn Leu Thr Ala Asn Gly Gly Val Val Gly Thr Asn Tyr Ser
    530             535             540

Asp Gly Ala Asn Tyr Asp Lys Ile Arg Thr Thr His Pro Ser Trp Ala
    545             550             555             560

Ile Tyr Gly Ser Glu Thr Ala Ser Ala Ile Asn Ser Arg Gly Ile Tyr
            565             570             575

Asn Arg Thr Thr Gly Gly Ala Gln Ser Ser Asp Lys Gln Leu Thr Ser
            580             585             590

Tyr Asp Asn Ser Ala Val Gly Trp Gly Ala Val Ala Ser Ser Ala Trp
        595             600             605

Tyr Asp Val Val Gln Arg Asp Phe Val Ala Gly Thr Tyr Val Trp Thr
    610             615             620

Gly Phe Asp Tyr Leu Gly Glu Pro Thr Pro Trp Asn Gly Thr Gly Ser
625             630             635             640

Gly Ala Val Gly Ser Trp Pro Ser Pro Lys Asn Ser Tyr Phe Gly Ile
            645             650             655

Val Asp Thr Ala Gly Phe Pro Lys Asp Thr Tyr Tyr Phe Tyr Gln Ser
            660             665             670

Gln Trp Asn Asp Asp Val His Thr Leu His Ile Leu Pro Ala Trp Asn
            675             680             685

Glu Asn Val Val Ala Lys Gly Ser Gly Asn Asn Val Pro Val Val Val
    690             695             700

Tyr Thr Asp Ala Ala Lys Val Lys Leu Tyr Phe Thr Pro Lys Gly Ser
705             710             715             720

Thr Glu Lys Arg Leu Ile Gly Glu Lys Ser Phe Thr Lys Lys Thr Thr
            725             730             735

Ala Ala Gly Tyr Thr Tyr Gln Val Tyr Glu Gly Ser Asp Lys Asp Ser
        740             745             750

Thr Ala His Lys Asn Met Tyr Leu Thr Trp Asn Val Pro Trp Ala Glu
        755             760             765

Gly Thr Ile Ser Ala Glu Ala Tyr Asp Glu Asn Asn Arg Leu Ile Pro
    770             775             780
```

```
Glu Gly Ser Thr Glu Gly Asn Ala Ser Val Thr Thr Thr Gly Lys Ala
785             790             795             800

Ala Lys Leu Lys Ala Asp Ala Asp Arg Lys Thr Ile Thr Ala Asp Gly
                805             810             815

Lys Asp Leu Ser Tyr Ile Glu Val Asp Val Thr Asp Ala Asn Gly His
                820             825             830

Ile Val Pro Asp Ala Ala Asn Arg Val Thr Phe Asp Val Lys Gly Ala
            835             840             845

Gly Lys Leu Val Gly Val Asp Asn Gly Ser Ser Pro Asp His Asp Ser
    850             855             860

Tyr Gln Ala Asp Asn Arg Lys Ala Phe Ser Gly Lys Val Leu Ala Ile
865             870             875             880

Val Gln Ser Thr Lys Glu Ala Gly Glu Ile Thr Val Thr Ala Lys Ala
                885             890             895

Asp Gly Leu Gln Ser Ser Thr Val Lys Ile Ala Thr Thr Ala Val Pro
            900             905             910

Gly Thr Ser Thr Glu Lys Thr Val Arg Ser Phe Tyr Tyr Ser Arg Asn
    915             920             925

Tyr Tyr Val Lys Thr Gly Asn Lys Pro Ile Leu Pro Ser Asp Val Glu
    930             935             940

Val Arg Tyr Ser Asp Gly Thr Ser Asp Arg Gln Asn Val Thr Trp Asp
945             950             955             960

Ala Val Ser Asp Asp Gln Ile Ala Lys Ala Gly Ser Phe Ser Val Ala
                965             970             975

Gly Thr Val Ala Gly Gln Lys Ile Ser Val Arg Val Thr Met Ile Asp
            980             985             990

Glu Ile Gly Ala Leu Leu Asn Tyr Ser Ala Ser Thr Pro Val Gly Thr
            995             1000            1005

Pro Ala Val Leu Pro Gly Ser Arg Pro Ala Val Leu Pro Asp Gly
    1010            1015            1020

Thr Val Thr Ser Ala Asn Phe Ala Val His Trp Thr Lys Pro Ala
```

EP 3 568 024 B1

1025                          1030                          1035

Asp Thr  Val Tyr Asn Thr Ala  Gly Thr Val Lys Val  Pro Gly Thr
    1040                 1045                 1050

Ala Thr  Val Phe Gly Lys Glu  Phe Lys Val Thr Ala  Thr Ile Arg
    1055                 1060                 1065

Val Gln  Arg Ser Gln Val Thr  Ile Gly Ser Ser Val  Ser Gly Asn
    1070                 1075                 1080

Ala Leu  Arg Leu Thr Gln Asn  Ile Pro Ala Asp Lys  Gln Ser Asp
    1085                 1090                 1095

Thr Leu  Asp Ala Ile Lys Asp  Gly Ser Thr Thr Val  Asp Ala Asn
    1100                 1105                 1110

Thr Gly  Gly Gly Ala Asn Pro  Ser Ala Trp Thr Asn  Trp Ala Tyr
    1115                 1120                 1125

Ser Lys  Ala Gly His Asn Thr  Ala Glu Ile Thr Phe  Glu Tyr Ala
    1130                 1135                 1140

Thr Glu  Gln Gln Leu Gly Gln  Ile Val Met Tyr Phe  Phe Arg Asp
    1145                 1150                 1155

Ser Asn  Ala Val Arg Phe Pro  Asp Ala Gly Lys Thr  Lys Ile Gln
    1160                 1165                 1170

Ile Ser  Ala Asp Gly Lys Asn  Trp Thr Asp Leu Ala  Ala Thr Glu
    1175                 1180                 1185

Thr Ile  Ala Ala Gln Glu Ser  Ser Asp Arg Val Lys  Pro Tyr Thr
    1190                 1195                 1200

Tyr Asp  Phe Ala Pro Val Gly  Ala Thr Phe Val Lys  Val Thr Val
    1205                 1210                 1215

Thr Asn  Ala Asp Thr Thr Thr  Pro Ser Gly Val Val  Cys Ala Gly
    1220                 1225                 1230

Leu Thr  Glu Ile Glu Leu Lys  Thr Ala Thr Ser Lys  Phe Val Thr
    1235                 1240                 1245

Asn Thr  Ser Ala Ala Leu Ser  Ser Leu Thr Val Asn  Gly Thr Lys
    1250                 1255                 1260

50

Val Ser Asp Ser Val Leu Ala   Ala Gly Ser Tyr Asn   Thr Pro Ala
    1265             1270               1275

Ile Ile Ala Asp Val Lys Ala   Glu Gly Glu Gly Asn   Ala Ser Val
    1280             1285               1290

Thr Val Leu Pro Ala His Asp   Asn Val Ile Arg Val   Ile Thr Glu
    1295             1300               1305

Ser Glu Asp His Val Thr Arg   Lys Thr Phe Thr Ile   Asn Leu Gly
    1310             1315               1320

Thr Glu Gln Glu Phe Pro Ala   Asp Ser Asp Glu Arg   Asp Tyr Pro
    1325             1330               1335

Ala Ala Asp Met Thr Val Thr   Val Gly Ser Glu Gln   Thr Ser Gly
    1340             1345               1350

Thr Ala Thr Glu Gly Pro Lys   Lys Phe Ala Val Asp   Gly Asn Thr
    1355             1360               1365

Ser Thr Tyr Trp His Ser Asn   Trp Thr Pro Thr Thr   Val Asn Asp
    1370             1375               1380

Leu Trp Ile Ala Phe Glu Leu   Gln Lys Pro Thr Lys   Leu Asp Ala
    1385             1390               1395

Leu Arg Tyr Leu Pro Arg Pro   Ala Gly Ser Lys Asn   Gly Ser Val
    1400             1405               1410

Thr Glu Tyr Lys Val Gln Val   Ser Asp Asp Gly Thr   Asn Trp Thr
    1415             1420               1425

Asp Ala Gly Ser Gly Thr Trp   Thr Thr Asp Tyr Gly   Trp Lys Leu
    1430             1435               1440

Ala Glu Phe Asn Gln Pro Val   Thr Thr Lys His Val   Arg Leu Lys
    1445             1450               1455

Ala Val His Thr Tyr Ala Asp   Ser Gly Asn Asp Lys   Phe Met Ser
    1460             1465               1470

Ala Ser Glu Ile Arg Leu Arg   Lys Ala Val Asp Thr   Thr Asp Ile
    1475             1480               1485

Ser Gly Ala Thr Val Thr Val   Pro Ala Lys Leu Thr   Val Asp Arg
    1490             1495               1500

```
Val Asp  Ala Asp His Pro Ala  Thr Phe Ala Thr Lys  Asp Val Thr
    1505             1510              1515
```

```
Val Thr  Leu Gly Asp Ala Thr  Leu Arg Tyr Gly Val  Asp Tyr Leu
    1520             1525              1530
```

```
Leu Asp  Tyr Ala Gly Asn Thr  Ala Val Gly Lys Ala  Thr Val Thr
    1535             1540              1545
```

```
Val Arg  Gly Ile Asp Lys Tyr  Ser Gly Thr Val Ala  Lys Thr Phe
    1550             1555              1560
```

```
Thr Ile  Glu Leu Lys Asn Ala  Pro Ala Pro Glu Pro  Thr Leu Thr
    1565             1570              1575
```

```
Ser Val  Ser Val Lys Thr Lys  Pro Ser Lys Leu Thr  Tyr Val Val
    1580             1585              1590
```

```
Gly Asp  Ala Phe Asp Pro Ala  Gly Leu Val Leu Gln  His Asp Arg
    1595             1600              1605
```

```
Gln Ala  Asp Arg Pro Pro Gln  Pro Leu Val Gly Glu  Gln Ala Asp
    1610             1615              1620
```

```
Glu Arg  Gly Leu Thr Cys Gly  Thr Arg Cys Asp Arg  Val Glu Gln
    1625             1630              1635
```

```
Leu Arg  Lys His Glu Asn Arg  Glu Ala His Arg Thr  Gly Leu Asp
    1640             1645              1650
```

```
His Leu  Glu Phe Val Gly Ala  Ala Asp Gly Ala Val  Gly Glu Gln
    1655             1660              1665
```

```
Ala Thr  Phe Lys Val His Val  His Ala Asp Gln Gly  Asp Gly Arg
    1670             1675              1680
```

```
His Asp  Asp Ala Asp Glu Arg  Asp Ile Asp Pro His  Val Pro Val
    1685             1690              1695
```

```
Asp His  Ala Val Gly Glu Leu  Ala Arg Ala Ala Cys  His His Val
    1700             1705              1710
```

```
Ile Gly  Leu Arg Val Asp Thr  His Arg Leu Lys Ala  Ser Gly Phe
    1715             1720              1725
```

```
Gln Ile  Pro Ala Asp Asp Met  Ala Glu Ile Asp Arg  Ile Thr Gly
    1730             1735              1740
```

52

```
                Phe His  Arg Phe Glu Arg His  Val Gly
                    1745                  1750
```

<210> 4
<211> 1935
<212> PRT
<213> Bifidobacterium bifidum

<400> 4

```
            Met Ala Val Arg Arg Leu Gly Gly Arg Ile Val Ala Phe Ala Ala Thr
            1               5               10              15

            Val Ala Leu Ser Ile Pro Leu Gly Leu Leu Thr Asn Ser Ala Trp Ala
                        20              25              30

            Val Glu Asp Ala Thr Arg Ser Asp Ser Thr Thr Gln Met Ser Ser Thr
                        35              40              45

            Pro Glu Val Val Tyr Ser Ser Ala Val Asp Ser Lys Gln Asn Arg Thr
                        50              55              60

            Ser Asp Phe Asp Ala Asn Trp Lys Phe Met Leu Ser Asp Ser Val Gln
            65                  70              75                  80

            Ala Gln Asp Pro Ala Phe Asp Asp Ser Ala Trp Gln Gln Val Asp Leu
                            85              90                  95

            Pro His Asp Tyr Ser Ile Thr Gln Lys Tyr Ser Gln Ser Asn Glu Ala
                        100             105             110

            Glu Ser Ala Tyr Leu Pro Gly Gly Thr Gly Trp Tyr Arg Lys Ser Phe
                        115             120             125

            Thr Ile Asp Arg Asp Leu Ala Gly Lys Arg Ile Ala Ile Asn Phe Asp
                        130             135             140

            Gly Val Tyr Met Asn Ala Thr Val Trp Phe Asn Gly Val Lys Leu Gly
            145             150             155             160

            Thr His Pro Tyr Gly Tyr Ser Pro Phe Ser Phe Asp Leu Thr Gly Asn
                            165             170             175

            Ala Lys Phe Gly Gly Glu Asn Thr Ile Val Val Lys Val Glu Asn Arg
                        180             185             190

            Leu Pro Ser Ser Arg Trp Tyr Ser Gly Ser Gly Ile Tyr Arg Asp Val
                        195             200             205
```

```
Thr Leu Thr Val Thr Asp Gly Val His Val Gly Asn Asn Gly Val Ala
    210             215             220

Ile Lys Thr Pro Ser Leu Ala Thr Gln Asn Gly Gly Asn Val Thr Met
225             230             235             240

Asn Leu Thr Thr Lys Val Ala Asn Asp Thr Lys Ala Ala Ala Asn Ile
            245             250             255

Thr Leu Lys Gln Thr Val Phe Pro Lys Gly Gly Lys Thr Asp Ala Ala
            260             265             270

Ile Gly Thr Val Thr Thr Ala Ser Lys Ser Ile Ala Ala Gly Ala Ser
        275             280             285

Ala Asp Val Thr Ser Thr Ile Thr Ala Ala Ser Pro Lys Leu Trp Ser
    290             295             300

Ile Lys Asn Pro Asn Leu Tyr Thr Val Arg Thr Glu Val Leu Asn Gly
305             310             315             320

Gly Lys Val Leu Asp Thr Tyr Asp Thr Glu Tyr Gly Phe Arg Trp Thr
            325             330             335

Gly Phe Asp Ala Thr Ser Gly Phe Ser Leu Asn Gly Glu Lys Val Lys
            340             345             350

Leu Lys Gly Val Ser Met His His Asp Gln Gly Ser Leu Gly Ala Val
            355             360             365

Ala Asn Arg Arg Ala Ile Glu Arg Gln Val Glu Ile Leu Gln Lys Met
    370             375             380

Gly Val Asn Ser Ile Arg Thr Thr His Asn Pro Ala Ala Lys Ala Leu
385             390             395             400

Ile Asp Val Cys Asn Glu Lys Gly Val Leu Val Val Glu Glu Val Phe
            405             410             415

Asp Met Trp Asn Arg Ser Lys Asn Gly Asn Thr Glu Asp Tyr Gly Lys
            420             425             430

Trp Phe Gly Gln Ala Ile Ala Gly Asp Asn Ala Val Leu Gly Gly Asp
            435             440             445

Lys Asp Glu Thr Trp Ala Lys Phe Asp Leu Thr Ser Thr Ile Asn Arg
```

450                    455                         460

Asp Arg Asn Ala Pro Ser Val Ile Met Trp Ser Leu Gly Asn Glu Met
465             470             475                 480

Met Glu Gly Ile Ser Gly Ser Val Ser Gly Phe Pro Ala Thr Ser Ala
                485             490                 495

Lys Leu Val Ala Trp Thr Lys Ala Ala Asp Ser Thr Arg Pro Met Thr
            500             505             510

Tyr Gly Asp Asn Lys Ile Lys Ala Asn Trp Asn Glu Ser Asn Thr Met
            515             520             525

Gly Asp Asn Leu Thr Ala Asn Gly Gly Val Val Gly Thr Asn Tyr Ser
        530             535             540

Asp Gly Ala Asn Tyr Asp Lys Ile Arg Thr Thr His Pro Ser Trp Ala
545             550             555                 560

Ile Tyr Gly Ser Glu Thr Ala Ser Ala Ile Asn Ser Arg Gly Ile Tyr
                565             570                 575

Asn Arg Thr Thr Gly Gly Ala Gln Ser Ser Asp Lys Gln Leu Thr Ser
        580             585             590

Tyr Asp Asn Ser Ala Val Gly Trp Gly Ala Val Ala Ser Ser Ala Trp
        595             600             605

Tyr Asp Val Val Gln Arg Asp Phe Val Ala Gly Thr Tyr Val Trp Thr
    610             615             620

Gly Phe Asp Tyr Leu Gly Glu Pro Thr Pro Trp Asn Gly Thr Gly Ser
625             630             635                 640

Gly Ala Val Gly Ser Trp Pro Ser Pro Lys Asn Ser Tyr Phe Gly Ile
            645             650             655

Val Asp Thr Ala Gly Phe Pro Lys Asp Thr Tyr Tyr Phe Tyr Gln Ser
        660             665             670

Gln Trp Asn Asp Asp Val His Thr Leu His Ile Leu Pro Ala Trp Asn
    675             680             685

Glu Asn Val Val Ala Lys Gly Ser Gly Asn Asn Val Pro Val Val Val
    690             695             700

```
Tyr Thr Asp Ala Ala Lys Val Lys Leu Tyr Phe Thr Pro Lys Gly Ser
705             710         715             720

Thr Glu Lys Arg Leu Ile Gly Glu Lys Ser Phe Thr Lys Lys Thr Thr
                725             730             735

Ala Ala Gly Tyr Thr Tyr Gln Val Tyr Glu Gly Ala Asp Lys Asp Ser
            740             745             750

Thr Ala His Lys Asn Met Tyr Leu Thr Trp Asn Val Pro Trp Ala Glu
            755             760             765

Gly Thr Ile Ser Ala Glu Ala Tyr Asp Glu Asn Asn Arg Leu Ile Pro
    770             775             780

Glu Gly Ser Thr Glu Gly Asn Ala Ser Val Thr Thr Thr Gly Lys Ala
785             790             795             800

Ala Lys Leu Lys Ala Asp Ala Asp Arg Lys Thr Ile Thr Ala Asp Gly
                805             810             815

Lys Asp Leu Ser Tyr Ile Glu Val Asp Val Thr Asp Ala Asn Gly His
            820             825             830

Ile Val Pro Asp Ala Ala Asn Arg Val Thr Phe Asp Val Lys Gly Ala
        835             840             845

Gly Lys Leu Val Gly Val Asp Asn Gly Ser Ser Pro Asp His Asp Ser
    850             855             860

Tyr Gln Ala Asp Asn Arg Lys Ala Phe Ser Gly Lys Val Leu Ala Ile
865             870             875             880

Val Gln Ser Thr Lys Glu Ala Gly Glu Ile Thr Val Thr Ala Lys Ala
            885             890             895

Asp Gly Leu Gln Ser Ser Thr Val Lys Ile Ala Thr Thr Ala Val Pro
        900             905             910

Gly Thr Ser Thr Glu Lys Thr Val Arg Ser Phe Tyr Tyr Ser Arg Asn
    915             920             925

Tyr Tyr Val Lys Thr Gly Asn Lys Pro Ile Leu Pro Ser Asp Val Glu
    930             935             940

Val Arg Tyr Ser Asp Gly Thr Ser Asp Arg Gln Asn Val Thr Trp Asp
945             950             955             960
```

56

```
Ala Val Ser Asp Asp Gln Ile Ala Lys Ala Gly Ser Phe Ser Val Ala
                965             970             975

Gly Thr Val Ala Gly Gln Lys Ile Ser Val Arg Val Thr Met Ile Asp
                980             985             990

Glu Ile Gly Ala Leu Leu Asn Tyr  Ser Ala Ser Thr Pro  Val Gly Thr
        995             1000            1005

Pro Ala  Val Leu Pro Gly Ser  Arg Pro Ala Val Leu  Pro Asp Gly
    1010            1015            1020

Thr Val  Thr Ser Ala Asn Phe  Ala Val Asp Trp Thr  Lys Pro Ala
    1025            1030            1035

Asp Thr  Val Tyr Asn Thr Ala  Gly Thr Val Lys Val  Pro Gly Thr
    1040            1045            1050

Ala Thr  Val Phe Gly Lys Glu  Phe Lys Val Thr Ala  Thr Ile Arg
    1055            1060            1065

Val Gln  Arg Ser Gln Val Thr  Ile Gly Ser Ser Val  Ser Gly Asn
    1070            1075            1080

Ala Leu  Arg Leu Thr Gln Asn  Ile Pro Ala Asp Lys  Gln Ser Asp
    1085            1090            1095

Thr Leu  Asp Ala Ile Lys Asp  Gly Ser Thr Thr Val  Asp Ala Asn
    1100            1105            1110

Thr Gly  Gly Gly Ala Asn Pro  Ser Ala Trp Thr Asn  Trp Ala Tyr
    1115            1120            1125

Ser Lys  Ala Gly His Asn Thr  Ala Glu Ile Thr Phe  Glu Tyr Ala
    1130            1135            1140

Thr Glu  Gln Gln Leu Gly Gln  Ile Val Met Tyr Phe  Phe Arg Asp
    1145            1150            1155

Ser Asn  Ala Val Arg Phe Pro  Asp Ala Gly Lys Thr  Lys Ile Gln
    1160            1165            1170

Ile Ser  Ala Asp Gly Lys Asn  Trp Thr Asp Leu Ala  Ala Thr Glu
    1175            1180            1185

Thr Ile  Ala Ala Gln Glu Ser  Ser Asp Arg Val Lys  Pro Tyr Thr
    1190            1195            1200
```

```
Tyr Asp Phe Ala Pro Val Gly Ala Thr Phe Val Lys Val Thr Val
    1205             1210             1215

Thr Asn Ala Asp Thr Thr Thr Pro Ser Gly Val Val Cys Ala Gly
    1220             1225             1230

Leu Thr Glu Ile Glu Leu Lys Thr Ala Thr Ser Lys Phe Val Thr
    1235             1240             1245

Asn Thr Ser Ala Ala Leu Ser Ser Leu Thr Val Asn Gly Thr Lys
    1250             1255             1260

Val Ser Asp Ser Val Leu Ala Ala Gly Ser Tyr Asn Thr Pro Ala
    1265             1270             1275

Ile Ile Ala Asp Val Lys Ala Glu Gly Glu Gly Asn Ala Ser Val
    1280             1285             1290

Thr Val Leu Pro Ala His Asp Asn Val Ile Arg Val Ile Thr Glu
    1295             1300             1305

Ser Glu Asp His Val Thr Arg Lys Thr Phe Thr Ile Asn Leu Gly
    1310             1315             1320

Thr Glu Gln Glu Phe Pro Ala Asp Ser Asp Glu Arg Asp Tyr Pro
    1325             1330             1335

Ala Ala Asp Met Thr Val Thr Ala Gly Ser Glu Gln Thr Ser Gly
    1340             1345             1350

Thr Ala Thr Glu Gly Pro Lys Lys Phe Ala Val Asp Gly Asn Thr
    1355             1360             1365

Ser Thr Tyr Trp His Ser Asn Trp Thr Pro Thr Thr Val Asn Asp
    1370             1375             1380

Leu Trp Ile Ala Phe Glu Leu Gln Lys Pro Thr Lys Leu Asp Ala
    1385             1390             1395

Leu Arg Tyr Leu Pro Arg Pro Ala Gly Ser Lys Asn Gly Ser Val
    1400             1405             1410

Thr Glu Tyr Lys Val Gln Val Ser Asp Asp Gly Thr Asn Trp Thr
    1415             1420             1425

Asp Ala Gly Ser Gly Thr Trp Thr Thr Asp Tyr Gly Trp Lys Leu
```

```
        1430                    1435                    1440


Ala Glu  Phe Asn Gln Pro Val  Thr Thr Lys His Val  Arg Leu Lys
     1445                    1450                    1455


Ala Val  His Thr Tyr Ala Asp  Ser Gly Asn Asp Lys  Phe Met Ser
     1460                    1465                    1470


Ala Ser  Glu Ile Arg Leu Arg  Lys Ala Val Asp Thr  Thr Asp Ile
     1475                    1480                    1485


Ser Gly  Ala Thr Val Thr Val  Pro Ala Lys Leu Thr  Val Asp Arg
     1490                    1495                    1500


Val Asp  Ala Asp His Pro Ala  Thr Phe Ala Thr Lys  Asp Val Thr
     1505                    1510                    1515


Val Thr  Leu Gly Asp Ala Thr  Leu Arg Tyr Gly Val  Asp Tyr Leu
     1520                    1525                    1530


Leu Asp  Tyr Ala Gly Asn Thr  Ala Val Gly Lys Ala  Thr Val Thr
     1535                    1540                    1545


Val Arg  Gly Ile Asp Lys Tyr  Ser Gly Thr Val Ala  Lys Thr Phe
     1550                    1555                    1560


Thr Ile  Glu Leu Lys Asn Ala  Pro Ala Pro Glu Pro  Thr Leu Thr
     1565                    1570                    1575


Ser Val  Ser Val Lys Thr Lys  Pro Ser Lys Leu Thr  Tyr Val Val
     1580                    1585                    1590


Gly Asp  Ala Phe Asp Pro Ala  Gly Leu Val Leu Gln  Leu Asn Tyr
     1595                    1600                    1605


Asp Asp  Asp Ser Thr Gly Thr  Val Thr Trp Asn Thr  Gln Thr Ala
     1610                    1615                    1620


Gly Asp  Phe Thr Phe Lys Pro  Ala Leu Asp Ala Lys  Leu Lys Val
     1625                    1630                    1635


Thr Asp  Lys Thr Val Thr Val  Thr Tyr Gln Gly Lys  Ser Ala Val
     1640                    1645                    1650


Ile Asp  Ile Thr Val Ser Gln  Pro Ala Pro Thr Val  Ser Lys Thr
     1655                    1660                    1665
```

```
Asp Leu  Asp Lys Ala Ile Lys  Ala Ile Glu Ala Lys  Asn Pro Asp
    1670              1675              1680

Ser Ser  Lys Tyr Thr Ala Asp  Ser Trp Lys Thr Phe  Ala Asp Ala
    1685              1690              1695

Met Ala  His Ala Lys Ala Val  Ile Ala Asp Asp Ser  Ala Thr Gln
    1700              1705              1710

Gln Asp  Val Asp Lys Ala Leu  Lys Ala Leu Thr Asp  Ala Tyr Ala
    1715              1720              1725

Gly Leu  Thr Glu Lys Thr Pro  Glu Pro Ala Pro Val  Ser Lys Ser
    1730              1735              1740

Glu Leu  Asp Lys Lys Ile Lys  Ala Ile Glu Ala Glu  Lys Leu Asp
    1745              1750              1755

Gly Ser  Lys Tyr Thr Ala Glu  Ser Trp Lys Ala Phe  Glu Thr Ala
    1760              1765              1770

Leu Ala  His Ala Lys Ala Val  Ile Ala Ser Asp Ser  Ala Thr Gln
    1775              1780              1785

Gln Asp  Val Asp Ala Ala Leu  Gly Ala Leu Thr Ser  Ala Arg Asp
    1790              1795              1800

Gly Leu  Thr Glu Lys Gly Glu  Val Lys Pro Asp Pro  Lys Pro Glu
    1805              1810              1815

Pro Gly  Thr Val Asp Lys Ala  Ala Leu Asp Lys Ala  Val Lys Lys
    1820              1825              1830

Val Glu  Ala Glu Lys Leu Asp  Gly Ser Lys Tyr Thr  Ala Asp Ser
    1835              1840              1845

Trp Lys  Ala Phe Glu Thr Ala  Leu Ala His Ala Lys  Ala Val Ile
    1850              1855              1860

Gly Asn  Ala Asn Ser Thr Gln  Phe Asp Ile Asp Asn  Ala Leu Ser
    1865              1870              1875

Met Leu  Asn Asp Ala Arg Ala  Ala Leu Lys Glu Lys  Pro Gly Arg
    1880              1885              1890

Ile Ile  Ala Ile Ile Asp Gly  Gly Ala Leu Ser Lys  Thr Gly Ala
    1895              1900              1905
```

```
Ser Val  Ala Ile Ile Ala Ser  Val Ala Ala Ala Met  Lys Ala Val
    1910                 1915                 1920


Gly Ala  Gly Val Met Ala Leu  Arg Pro Pro Lys Trp
    1925                 1930                 1935
```